# EUROPEAN PATENT APPLICATION

(11) **EP 4 821 518 A1**
(43) Date of publication of application: **14.10.2026**
(21) Application number: 24903554.4
(22) Date of filing: 03.12.2024
(51) Int. Cl.: A61M 1/36, A61M 60/113, A61M 60/268, A61M 60/37, A61M 60/427

(54) **MEDICINAL LIQUID SUPPLY DEVICE AND MEDICINAL LIQUID DILUTION METHOD**

(30) Priority: 12.12.2023 JP 2023209695
(71) Applicant: Nikkiso Co., Ltd., Tokyo 150-6022 (JP)
(72) Inventor: KAWARABAYASHI, Satoru, Tokyo 150-6022 (JP); YAMAMURA, Masaru, Tokyo 150-6022 (JP)
(74) Representative: von Hirschhausen, Helge
(86) International application number: PCT/JP2024/042639
(87) International publication number: WO 2025/126896

(57) **Abstract**

Provided is a medicinal liquid supply apparatus which is less limited by a packaging material of a medicinal liquid such as heparin, and can be used for treatment to be performed a plurality of times with the medicinal liquid in a unit of packaging. The medicinal liquid supply apparatus supplies a medicinal liquid to a blood circuit in a blood purification apparatus that purifies blood, and includes a composition-holding portion into which an anticoagulant contained in a container can be introduced from the container and which holds a composition containing at least the introduced anticoagulant; and a connecting portion that connects the composition-holding portion to the blood circuit, and allows the composition to be led out from the composition-holding portion to the blood circuit.

## Description

### TECHNICAL FIELD

The present invention relates to a medicinal liquid supply apparatus and a medicinal liquid dilution method.

### BACKGROUND ART

Heparin may be used as an anticoagulant for blood in a dialysis device or the like. Generally, heparin is delivered while being contained in a vial or the like. In a system using heparin contained in a vial, by setting the vial in a device, heparin can be used immediately from the vial (see, for example, Patent Literature 1).

### CITATION LIST

### Patent Literature

Patent Literature 1: JP 2021-118944 A

### SUMMARY OF INVENTION

### Technical Problem

In a conventional system, a vial is set in a device. For this reason, only a vial having a certain shape and size that can be used for the system has to be used. For this reason, a packaging material of heparin is limited to a vial. Furthermore, only treatment using heparin in a unit of packaging can be performed, and it is difficult to perform treatment a plurality of times.

The present invention has been made in view of the above point. An object of the present invention is to provide a medicinal liquid supply apparatus which is less limited by a packaging material of a medicinal liquid such as heparin, and can be used for treatment to be performed a plurality of times with the medicinal liquid in a unit of packaging.

### SOLUTION TO PROBLEM

A medicinal liquid supply apparatus according to the present invention is a medicinal liquid supply apparatus that supplies a medicinal liquid to a blood circuit in a blood purification apparatus that purifies blood. The medicinal liquid supply apparatus comprises a composition-holding portion into which an anticoagulant contained in a container can be introduced from the container and which holds a composition containing at least the introduced anticoagulant and a connecting portion that connects the composition-holding portion to the blood circuit, and allows the composition to be led out from the composition-holding portion to the blood circuit.

### ADVANTAGEOUS EFFECTS OF INVENTION

The medicinal liquid supply apparatus is less limited by a packaging material of a medicinal liquid such as heparin, and can be used for treatment to be performed a plurality of times with the medicinal liquid in a unit of packaging.

### BRIEF DESCRIPTION OF DRAWINGS

Fig. 1 is a schematic diagram illustrating a configuration of a medicinal liquid supply apparatus according to a first embodiment.
Fig. 2 is a schematic diagram illustrating a filling step in the medicinal liquid supply apparatus according to the first embodiment.
Fig. 3 is a schematic diagram illustrating a dilution step in the medicinal liquid supply apparatus according to the first embodiment.
Fig. 4 is a schematic diagram illustrating an administration preparation step in the medicinal liquid supply apparatus according to the first embodiment.
Fig. 5 is a schematic diagram illustrating a dilution step (a) and an administration preparation step (b) in a medicinal liquid supply apparatus according to a second embodiment.
Fig. 6 is a schematic diagram illustrating a configuration of a medicinal liquid supply apparatus according to a third embodiment.
Fig. 7 is a schematic diagram illustrating a filling step in the medicinal liquid supply apparatus according to the third embodiment.
Fig. 8 is a schematic diagram illustrating a dilution step in the medicinal liquid supply apparatus according to the third embodiment.
Fig. 9 is a schematic diagram illustrating an administration preparation step in the medicinal liquid supply apparatus according to the third embodiment.
Fig. 10 is a functional block diagram illustrating functions of a control device 66.
Fig. 11 is a flowchart illustrating overall processing of the control device 66.

### Description of Embodiments.

### DESCRIPTION OF EMBODIMENTS

### <<<<<Outline of Present Embodiment>>>>>

Conventionally, heparin is used as an anticoagulant for blood in dialysis treatment. Heparin is often delivered while being contained in a vial or filled in a syringe.

When heparin contained in a vial is used, the vial is directly set in a device and heparin is administered. However, it is difficult to use a packaging material other than a vial corresponding to the device. In addition, in a general vial, it is necessary to completely use the entire amount of contained heparin in one treatment, and it is difficult to use heparin for treatment to be performed a plurality of times.

A packaging material such as a vial often depends on a device in terms of the size and shape of the packaging material, and there are few options for a packaging material that can be used.

As described above, there has been a demand for a device and a system that increase the number of options for a packaging material such that desired heparin can be used, and that can use the packaging material a plurality of times.

### <<<<<Details of present embodiment>>>>>

Hereinafter, first to third embodiments will be described with reference to the drawings.

### <<Direction>>

### <Up direction, upper side, upward, above, and the like>

An up direction is a direction opposite to a down direction described later, and is a direction opposite to the direction of gravity.

### <Down direction, lower side, downward, below, and the like>

A down direction is the direction of gravity. That is, the down direction is a direction indicated by a yarn from which an object is suspended.

### <Up-down direction>

An up-down direction is a direction along the up direction and the down direction. An orientation of the up-down direction is not limited as long as the up-down direction is along the up direction and the down direction.

### <Longitudinal direction>

In a member or the like having a long shape, a longitudinal direction refers to a direction in which the member extends long. The longitudinal direction refers to a direction in which the longest portion or region extends.

### <Short direction>

In a member or the like having a long shape, a short direction refers to a direction extending short, for example, a direction perpendicular to a longitudinal direction. The short direction refers to a direction in which the shortest portion or region extends.

### <<<<First embodiment>>>>

### <<<Configuration of blood purification apparatus 70>>>

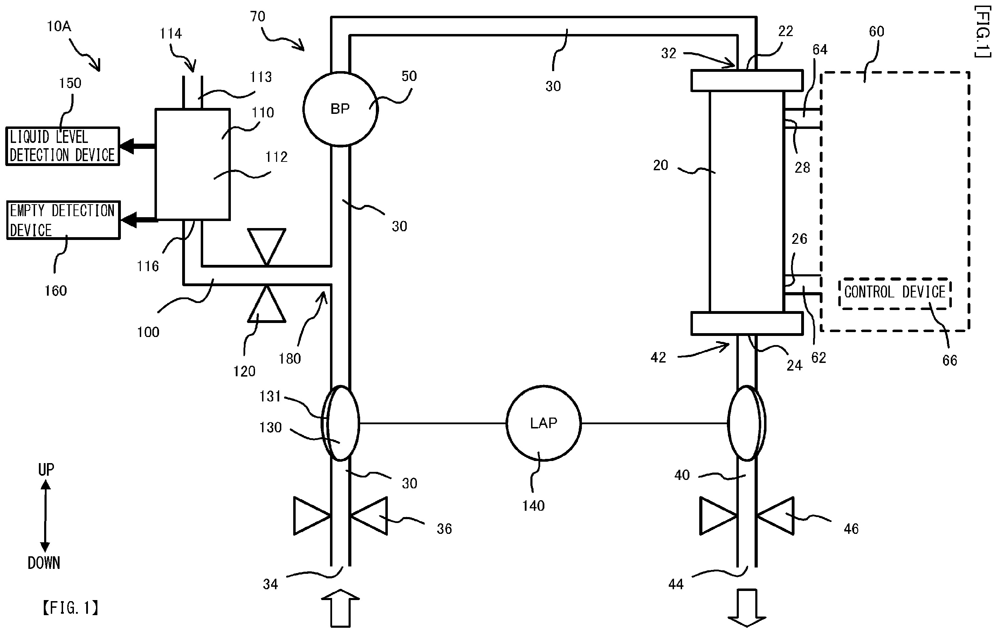

Fig. 1 is a schematic diagram illustrating a configuration of a medicinal liquid supply apparatus 10A according to a first embodiment. The medicinal liquid supply apparatus 10A of the first embodiment is connected to a blood purification apparatus 70 and applied to a hemodialysis apparatus.

The blood purification apparatus 70 mainly comprises:
a dialyzer 20 having a blood purification function;
a blood circuit to which an arterial side blood circuit 30 and a venous side blood circuit 40 are connected;
a blood pump 50;
a dialysis device main body 60 to which a dialysate introduction line 62 and a dialysate discharge line 64 are connected;
a control device 66;
a medicinal liquid clamping means 120;
a diaphragm pump 130;
a liquid level adjusting pump 140;
a liquid level detection device 150; and
an empty detection device 160.

### <Dialyzer 20>

The dialyzer 20 has a blood purification membrane (not illustrated). The blood purification membrane may be a hollow fiber type hemodialysis membrane or hemodiafiltration membrane, or a flat membrane type hemodialysis membrane. The dialyzer 20 has a blood inlet 22 and a blood outlet 24. The blood inlet 22 is an opening through which blood is introduced into the dialyzer 20. The blood outlet 24 is an opening through which blood introduced into the dialyzer 20 is led out. Furthermore, the dialyzer 20 has a dialysate inlet 26 and a dialysate discharge port 28. The dialysate inlet 26 is an opening through which a dialysate is introduced into the dialyzer 20. The dialysate discharge port 28 is an opening through which a dialysate introduced into the dialyzer 20 is discharged. The dialyzer 20 brings blood introduced from the blood inlet 22 into contact with a dialysate via a blood purification membrane to purify the blood.

### <Arterial side blood circuit 30>

The arterial side blood circuit 30 mainly has a flexible and long tube. The arterial side blood circuit 30 has a first end 32 and a second end 34. The first end 32 of the arterial side blood circuit 30 is connected to the blood inlet 22 of the dialyzer 20. Blood collected from a patient's blood vessel is guided from the first end 32 of the arterial side blood circuit 30 into the blood purification membrane of the dialyzer 20 via the blood inlet 22 of the dialyzer 20. A connector (not illustrated) for attaching an arterial side puncture needle (not illustrated) can be disposed at the second end 34 of the arterial side blood circuit 30.

### <Venous side blood circuit 40>

Similar to the arterial side blood circuit 30, the venous side blood circuit 40 mainly has a flexible and long tube. The venous side blood circuit 40 has a first end 42 and a second end 44, with an air trap chamber (not illustrated) in the middle. The first end 42 of the venous side blood circuit 40 is connected to the blood outlet 24 of the dialyzer 20. Blood that has passed through the blood purification membrane is led out from the blood outlet 24 of the dialyzer 20. A connector (not illustrated) for attaching a venous side puncture needle (not illustrated) can be disposed at the second end 44 of the venous side blood circuit 40.

### <Blood pump 50>

The blood pump 50 is disposed between the first end 32 and the second end 34 of the arterial side blood circuit 30. The blood pump 50 is constituted by a tube pump. The tube pump has a rotatable roller (not illustrated). By pressing and crushing the tube by the rotating roller, blood and a priming liquid in the tube of the arterial side blood circuit 30 are caused to flow.

### <Extracorporeal circulation>

Patient's blood collected by the arterial side puncture needle reaches the dialyzer 20 via the arterial side blood circuit 30, is purified, then flows through the venous side blood circuit 40, and returns to the patient's body via the venous side puncture needle. As a result, extracorporeal circulation is performed.

### <Arterial side and venous side>

A puncture needle side at which blood is removed (collected) is referred to as an "arterial side", and a puncture needle side at which blood is returned is referred to as a" venous side". Therefore, whether a side is referred to as the "arterial side" or the "venous side" is not determined depending on whether a blood vessel to be punctured is an artery or a vein.

### <Arterial clamping means 36 and venous clamping means 46>

An arterial clamping means 36 capable of opening and closing a flow path of the arterial side blood circuit 30 is disposed at a distal end of the arterial side blood circuit 30. A venous clamping means 46 capable of opening and closing a flow path of the venous side blood circuit 40 is disposed at a distal end of the venous side blood circuit 40. The arterial clamping means 36 and the venous clamping means 46 are controlled to an open state or a closed state by a control signal output from the control device 66.

### <Dialysate introduction line 62 and dialysate discharge line 64>

The dialysate introduction line 62 is connected to the dialysate inlet 26 of the dialyzer 20. The dialysate discharge line 64 is connected to the dialysate discharge port 28 of the dialyzer 20. A dialysate can be introduced into the dialyzer 20 via the dialysate introduction line 62 and discharged from the dialysate discharge line 64 through the outside of a hollow fiber membrane. The inside of the hollow fiber membrane (purification membrane) included in the dialyzer 20 forms a blood flow path through which blood can flow, and the outside of the hollow fiber membrane forms a dialysate flow path through which a dialysate can flow.

### <Dialysis device main body 60>

The dialysate introduction line 62 and the dialysate discharge line 64 extend to the dialysis device main body 60. The dialysis device main body 60 includes a liquid feeding pump and a water removing pump (both not illustrated). The liquid feeding pump introduces a dialysate prepared to have a predetermined concentration into the dialyzer 20 and discharges the dialysate after dialysis from the dialyzer 20. The water removing pump removes moisture from blood flowing in the dialyzer 20.

### <Control device 66>

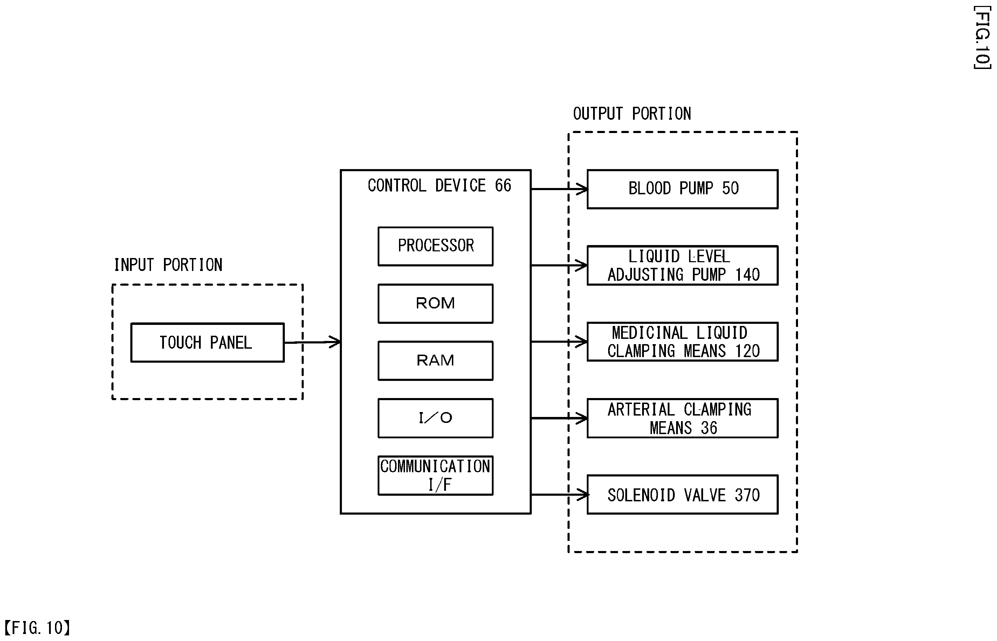

Fig. 10 is a functional block diagram illustrating overall functions of the dialysis device main body 60 including the control device 66. The dialysis device main body 60 includes an input portion, the control device 66, and an output portion.

The input portion is a device or a component that can be operated by an operator of the dialysis device main body 60, such as a touch panel or a keyboard. The operator can input data from the input portion or input an instruction for control. When detecting an input operation by the operator, the input portion outputs a detection signal corresponding to the input operation to the control device 66.

The control device 66 mainly includes a processor (such as a central processing unit (CPU)), a read only memory (ROM), a random access memory (RAM), an input/output interface (I/O), an interface device (I/F), an auxiliary storage device (such as a hard disk drive (HDD) or a solid state drive (SSD)), and the like. The ROM stores programs, constants, and the like for executing various types of processing such as control processing. The RAM temporarily stores values of variables and the like used when a program is executed.

The output portion mainly includes the blood pump 50, the liquid level adjusting pump 140, the medicinal liquid clamping means 120, the arterial clamping means 36, and a solenoid valve 370 (third embodiment described later). An operator operates the input portion, whereby the input portion can output a detection signal corresponding to the input operation to the control device 66, and the control device 66 can output a control signal to the output portion to control the output portion.

The control device 66 outputs various control signals and receives various detection signals as inputs via the I/O. For example, the control device 66 outputs a control signal to the medicinal liquid clamping means 120, the diaphragm pump 130, the liquid level adjusting pump 140, and the like described later. In addition, the control device 66 receives a detection signal as an input from the liquid level detection device 150, the empty detection device 160, and the like.

### <<Medicinal liquid clamping means 120>>

The medicinal liquid clamping means 120 is disposed in a medicinal liquid line 100 (described later) of the medicinal liquid supply apparatus 10A. The medicinal liquid clamping means 120 is in either an open state or a closed state. When the medicinal liquid clamping means 120 is in an open state, liquid can flow in the medicinal liquid line 100. When the medicinal liquid clamping means 120 is in a closed state, liquid cannot flow in the medicinal liquid line 100.

The medicinal liquid clamping means 120 includes a drive device (not illustrated) such as a solenoid or a motor. In the medicinal liquid clamping means 120, the drive device is driven by a control signal output from the control device 66, whereby the medicinal liquid clamping means 120 is brought into an open state or a closed state.

### <<Diaphragm pump 130>>

The diaphragm pump 130 has a deformable diaphragm 131. The diaphragm pump 130 controls a flow of liquid by deformation of the diaphragm 131. The diaphragm 131 of the diaphragm pump 130 is driven by the liquid level adjusting pump 140. The diaphragm pump 130 presses or sucks the diaphragm 131 by the liquid level adjusting pump 140. By deforming the diaphragm 131 by pressing or suction of the diaphragm 131 to generate a positive pressure or a negative pressure in the arterial side blood circuit 30 or the venous side blood circuit 40, a flow of liquid in the arterial side blood circuit 30 or the venous side blood circuit 40 is controlled. The diaphragm pump 130 can repeat pressing and suction by the liquid level adjusting pump 140.

In the first embodiment, the diaphragm pump 130 is disposed in each of the arterial side blood circuit 30 and the venous side blood circuit 40. In the first embodiment, the diaphragm pump 130 disposed in the arterial side blood circuit 30 is mainly used. The diaphragm pump 130 can cause liquid to flow in the arterial side blood circuit 30.

### <<Liquid level adjusting pump 140>>

The liquid level adjusting pump 140 deforms the diaphragm 131 of the diaphragm pump 130. The liquid level adjusting pump 140 presses or sucks the diaphragm 131 of the diaphragm pump 130.

The liquid level adjusting pump 140 includes a drive device (not illustrated) such as a solenoid or a motor. In the liquid level adjusting pump 140, the drive device is driven by a control signal output from the control device 66, whereby the liquid level adjusting pump 140 performs pressing or suction.

### <<Liquid level detection device 150>>

The liquid level detection device 150 can detect the amount of undiluted heparin or diluted heparin introduced into a pipette 110. The liquid level detection device 150 outputs a signal indicating a liquid level to the control device 66.

### <<Empty detection device 160>>

The empty detection device 160 can detect whether or not undiluted heparin or diluted heparin is present in the pipette 110. The empty detection device 160 outputs a signal indicating that heparin or diluted heparin is not present. By disposing the empty detection device 160, in an administration preparation step described later, diluted heparin can be led out until the pipette 110 becomes empty, and therefore waste due to residual medicinal liquid can be reduced. Furthermore, since lead-out of diluted heparin can be stopped when the pipette 110 becomes empty, a risk of air mixing into the arterial side blood circuit 30 can be reduced.

### <<<Drug and the like>>>

### <Heparin (anticoagulant)>

Heparin is used as an anticoagulant in dialysis treatment. Usually, heparin is delivered in a form of being enclosed in a syringe or the like.

### <Priming liquid (diluent)>

In dialysis treatment, physiological saline, a dialysate, or the like is used as a priming liquid. In dialysis treatment, heparin is diluted with a priming liquid.

### <<<Configuration of medicinal liquid supply apparatus 10A>>

The medicinal liquid supply apparatus 10A can be disposed outside the blood circuit, and can be used with less change to a conventional blood circuit.

As illustrated in Fig. 1, the medicinal liquid supply apparatus 10A mainly includes the medicinal liquid line 100; and the pipette 110.

### <<Medicinal liquid line 100>>

The medicinal liquid line 100 is connected to the blood circuit. Liquid can flow between the medicinal liquid line 100 and the blood circuit. Examples of the liquid include, but are not limited to, a priming liquid and diluted heparin. For example, the medicinal liquid line 100 is connected to and communicates with the arterial side blood circuit 30. The medicinal liquid line 100 is preferably constituted by a flexible tube or the like. The pipette 110 can be disposed at a position away from the arterial side blood circuit 30.

The medicinal liquid line 100 is connected to the arterial side blood circuit 30 at a connection portion 180. The medicinal liquid line 100 is preferably detachably connected at the connection portion 180. The medicinal liquid line 100 can be used as a disposable instrument.

### <<Pipette 110>>

Heparin and/or a priming liquid is introduced into the pipette 110. In the pipette 110, heparin is diluted with a priming liquid. A pipette is an instrument for containing, temporarily storing, and moving a small amount of liquid. The pipette may have a measuring scale. By using the measuring scale, a volume can be simply and easily measured. The pipette can be used as a disposable instrument. Note that any container may be used without being limited to the pipette as long as it can temporarily contain heparin and/or a priming liquid, and can lead out undiluted heparin or diluted heparin.

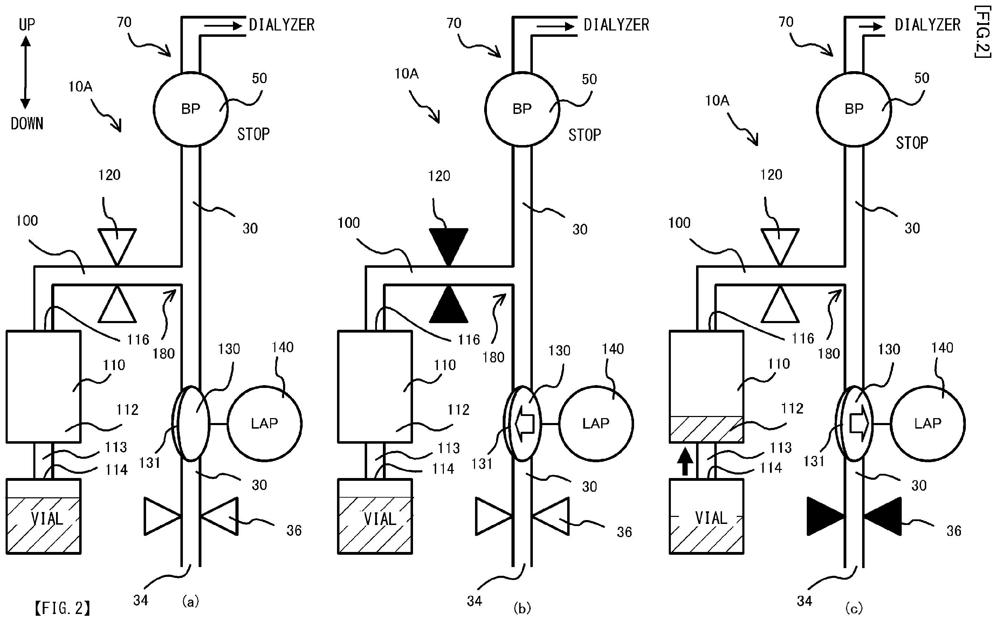

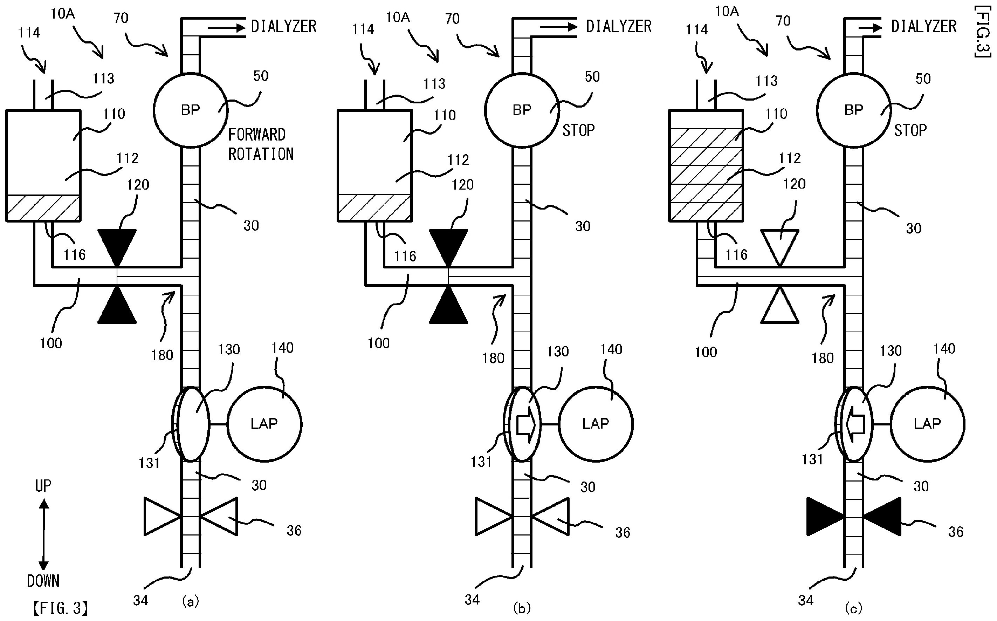

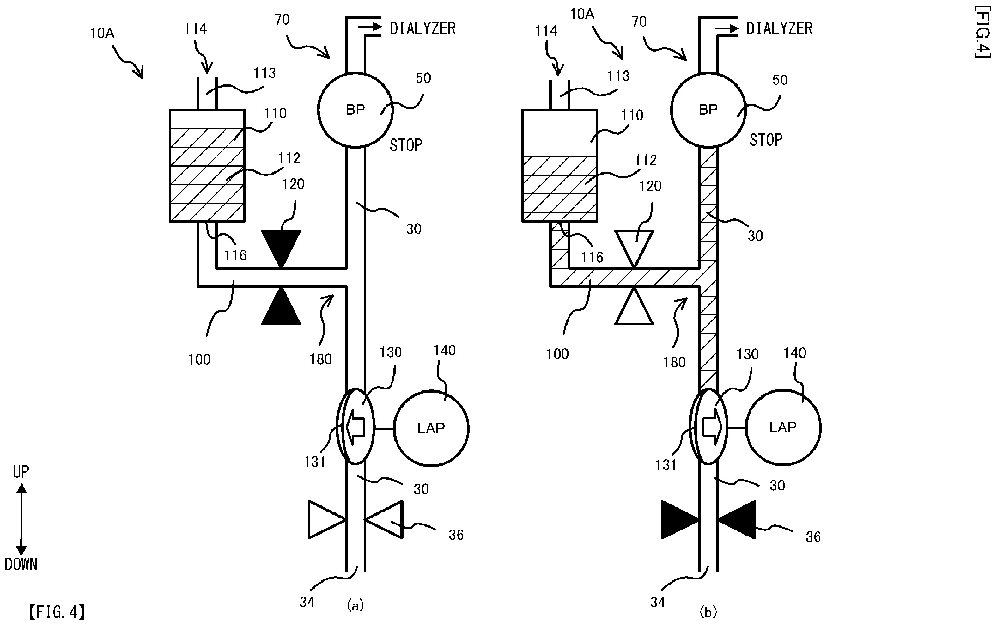

The pipette 110 has an overall long shape. The pipette 110 is disposed such that a longitudinal direction thereof is along the up-down direction. The pipette 110 is disposed so as to be turned upside down. The pipette 110 is in either an upright state or an inverted state. The upright state is a state in which an upper end opening 114 (described later) of the pipette 110 opens upward, and is a state of the pipette 110 in the administration preparation step (see Fig. 4) or the dilution step (see Fig. 3). The inverted state is a state in which the upper end opening 114 of the pipette 110 opens downward, and is a state of the pipette 110 in the filling step (see Fig. 2).

An operation of turning the pipette 110 upside down may be performed manually or by using a drive device (not illustrated) such as a motor. The control device 66 of the blood purification apparatus 70 outputs a control signal for turning the pipette 110 upside down to the drive device such as a motor. When the control device 66 of the blood purification apparatus 70 outputs an upright command signal, the pipette 110 is brought into an upright state by the drive device such as a motor. The control device 66 can determine that the state of the pipette 110 is the upright state by outputting the upright command signal. When the control device 66 of the blood purification apparatus 70 outputs an inverted command signal, the pipette 110 is brought into an inverted state by the drive device such as a motor. The control device 66 can determine that the state of the pipette 110 is the inverted state by outputting the inverted command signal.

In addition, a sensor (not illustrated) for detecting whether the pipette 110 is in the upright state or the inverted state may be disposed. The sensor may be any sensor as long as it detects the upright state or the inverted state of the pipette 110 and outputs a signal indicating a detection result, regardless of whether the sensor is an optical sensor, a mechanical sensor, or a sensor using another detection method. The control device 66 of the blood purification apparatus 70 can acquire the state of the pipette 110 by receiving a signal indicating a detection result, emitted from the sensor. Even when a failure occurs in the drive device such as a motor, the state of the pipette 110 can be accurately acquired.

Furthermore, when an operator manually turns the pipette 110 upside down, the operator operates a touch panel after an operation of turning the pipette 110 upside down is completed. For example, when the operator brings the pipette 110 into the upright state, the operator inputs, to the touch panel, information indicating that the pipette 110 has been brought into the upright state. By this operation, the touch panel outputs a signal indicating that the pipette 110 has been brought into the upright state to the control device. As a result, the control device 66 can determine that the state of the pipette 110 is the upright state. On the other hand, when the operator brings the pipette 110 into the inverted state, the operator inputs, to the touch panel, information indicating that the pipette 110 has been brought into the inverted state. By this operation, the touch panel outputs a signal indicating that the pipette 110 has been brought into the inverted state to the control device. As a result, the control device 66 can determine that the state of the pipette 110 is the inverted state.

The pipette 110 mainly has a containing portion 112, an extending portion 113, the upper end opening 114, and a lower end opening 116.

### <Containing portion 112>

The containing portion 112 has a long and substantially cylindrical shape. Heparin and/or a priming liquid is introduced into the containing portion 112. When the priming liquid is introduced into the containing portion 112, heparin is diluted with the priming liquid in the containing portion 112. When the priming liquid is not introduced into the containing portion 112, heparin is not diluted with the priming liquid in the containing portion 112, and heparin is simply contained therein.

### <Extending portion 113>

The extending portion 113 has a long and substantially cylindrical shape. The extending portion 113 extends in a direction away from the containing portion 112 above the containing portion 112. The extending portion 113 has a smaller diameter than the containing portion 112. By making the extending portion 113 thinner than the containing portion 112, the extending portion 113 can be easily inserted into and extracted from openings of various containers. By making the extending portion 113 compatible with containers having various shapes and sizes, contained heparin can be introduced into the pipette 110. In particular, the extending portion 113 is preferably formed to be thinner (tapered) as being away from the containing portion 112. The extending portion 113 can be more easily inserted into and extracted from openings of various containers.

The extending portion 113 is disposed concentrically with the containing portion 112. That is, the extending portion 113 is disposed such that a central axis of the extending portion 113 coincides with a central axis of the containing portion 112.

### <Upper end opening 114>

The extending portion 113 has the upper end opening 114 at an upper end thereof (a portion farthest from the containing portion 112). The upper end opening 114 communicates with the containing portion 112 and the extending portion 113. The upper end opening 114 can be connected to a vial (see Fig. 2). Heparin stored in the vial is introduced into the containing portion 112 from the upper end opening 114 via the extending portion 113.

In a normal upright state in which the pipette 110 is not turned upside down, the upper end opening 114 is directed upward. On the other hand, in an inverted state in which the pipette 110 is turned upside down, the upper end opening 114 is directed downward. Note that heparin is introduced into the containing portion 112 via the upper end opening 114 with the upper end opening 114 directed downward in an inverted state in which the pipette 110 is turned upside down.

### <Lower end opening 116>

The pipette 110 has the lower end opening 116 in a lower portion thereof. The lower end opening 116 communicates with the containing portion 112. The pipette 110 is connected to the medicinal liquid line 100 at the lower end opening 116. A priming liquid is introduced from the lower end opening 116 into the containing portion 112 via the medicinal liquid line 100. In addition, heparin diluted with the priming liquid is led out from the containing portion 112 to the medicinal liquid line 100 via the lower end opening 116. In a normal upright state in which the pipette 110 is not turned upside down, the lower end opening 116 is directed downward. Note that the priming liquid or diluted heparin is introduced or led out in a normal upright state in which the pipette 110 is not turned upside down, that is, in a state in which the lower end opening 116 is directed downward. In addition, heparin not diluted with the priming liquid is also led out in a normal upright state in which the pipette 110 is not turned upside down, that is, in a state in which the lower end opening 116 is directed downward.

### <<Another configuration of medicinal liquid supply apparatus 10A>>

The example has been described in which the medicinal liquid supply apparatus 10A mainly includes:
the medicinal liquid line 100; and
the pipette 110, and
the blood purification apparatus 70 mainly includes:
the dialyzer 20 having a blood purification function;
the blood circuit to which the arterial side blood circuit 30 and the venous side blood circuit 40 are connected;
the blood pump 50;
the dialysis device main body 60 to which the dialysate introduction line 62 and the dialysate discharge line 64 are connected;
the control device 66;
the medicinal liquid clamping means 120;
the diaphragm pump 130;
the liquid level adjusting pump 140;
the liquid level detection device 150; and
the empty detection device 160.

The present invention is not limited to this configuration, and the medicinal liquid supply apparatus 10A may include at least one of the medicinal liquid clamping means 120, the diaphragm pump 130, the liquid level adjusting pump 140, the liquid level detection device 150, and the empty detection device 160. For example, a control device (not illustrated) including a processor (processing device) or the like is disposed in the medicinal liquid supply apparatus 10A to make it possible to output a control signal from the control device or to input a detection signal to the control device. Furthermore, the control device of the medicinal liquid supply apparatus 10A is communicably connected to the control device 66 of the blood purification apparatus 70. Control is performed by appropriately dividing the medicinal liquid clamping means 120, the diaphragm pump 130, the liquid level adjusting pump 140, the liquid level detection device 150, and the empty detection device 160 into those controlled by the control device 66 of the blood purification apparatus 70 and those controlled by the control device of the medicinal liquid supply apparatus 10A.

Note that only the control device of the medicinal liquid supply apparatus 10A may control all of the medicinal liquid clamping means 120, the diaphragm pump 130, the liquid level adjusting pump 140, the liquid level detection device 150, and the empty detection device 160. The medicinal liquid clamping means 120, the diaphragm pump 130, and the liquid level adjusting pump 140 are controlled by a control signal output from the control device of the medicinal liquid supply apparatus 10A, and detection signals output from the liquid level detection device 150 and the empty detection device 160 are input to the control device of the medicinal liquid supply apparatus 10A.

### <<Operation performed by medicinal liquid supply apparatus 10A>>

Fig. 2 is a schematic diagram illustrating a filling step in the medicinal liquid supply apparatus 10A according to the first embodiment. Fig. 3 is a schematic diagram illustrating a dilution step in the medicinal liquid supply apparatus 10A according to the first embodiment. Fig. 4 is a schematic diagram illustrating an administration preparation step in the medicinal liquid supply apparatus 10A according to the first embodiment. As illustrated in Figs. 2 to 4, an operationperformed by the medicinal liquid supply apparatus 10A includes the filling step, the dilution step, and the administration preparation step.

In Figs. 2 to 4, a valve indicated in white indicates an open state, and a valve indicated in black indicates a closed state. In Figs. 2 to 4, presence of heparin is indicated by oblique lines, and presence of a priming liquid is indicated by horizontal lines. A leftward white arrow on the diaphragm pump 130 indicates that the diaphragm 131 has been pressed, and a rightward white arrow on the diaphragm pump 130 indicates that the diaphragm 131 has been sucked.

### <<Filling step>>

As illustrated in Fig. 2, the filling step is a step of introducing heparin from a vial into the pipette 110. By the filling step, heparin contained in a container such as a vial (such as a container at the time of delivery) can be introduced into the pipette 110. Heparin can be used without depending on the shape of the container or the form of the container, such as an opening of the container, at the time of delivery. It is compatible with a wide variety of containers in which heparin is contained. Note that, in the filling step, the blood pump 50 is stopped.

### <Filling step 1>

Fig. 2(a) illustrates a first step of the filling step. At this time, both the medicinal liquid clamping means 120 and the arterial clamping means 36 are in an open state (indicated in white).

First, a vial containing heparin is disposed below the pipette 110. Next, the pipette 110 is turned upside down to locate the upper end opening 114 on a lower side (inverted state). The pipette 110 may be turned upside down manually or by a control signal of the control device 66. By disposing a drive device such as a motor in the pipette 110, the pipette 110 can be turned upside down by the control signal. The upper end opening 114 located on a lower side is inserted into the vial to communicate with the vial.

### <Filling step 2>

Fig. 2(b) illustrates a second step of the filling step.

A processor of the control device 66 outputs a control signal to bring the medicinal liquid clamping means 120 into a closed state (indicated in black). The processor of the control device 66 outputs a control signal to drive the liquid level adjusting pump 140 to press the diaphragm 131 of the diaphragm pump 130.

### <Filling step 3>

Fig. 2(c) illustrates a last step of the filling step.

The processor of the control device 66 outputs a control signal to stop the blood pump 50 to bring the arterial clamping means 36 into a closed state (black). The processor of the control device 66 outputs a control signal to bring the medicinal liquid clamping means 120 into an open state (white).

The processor of the control device 66 outputs a control signal to drive the liquid level adjusting pump 140 to suck the diaphragm 131 of the diaphragm pump 130. As a result, the inside of the pipette 110 can be brought into a negative pressure state. The inside of the pipette 110 is brought into the negative pressure state, whereby heparin contained in the vial is sucked from the vial and introduced into the pipette 110.

An amount of heparin corresponding to a difference between deformation due to pressing of the diaphragm 131 of the diaphragm pump 130 and deformation due to suction of the diaphragm 131 of the diaphragm pump 130 is introduced into the pipette 110. By deforming the diaphragm 131 of the diaphragm pump 130 at least once, a desired amount of heparin can be introduced from the vial into the pipette 110.

By the filling step illustrated in Figs. 2(a) to 2(c), heparin can be introduced into the pipette 110 without depending on the shape of a container containing heparin or the form of the container, such as an opening.

After the filling step is completed, the pipette 110 is turned upside down (returned from the inverted state to the upright state), and the upper end opening 114 is located on an upper side. Similarly to the filling step, the pipette 110 may be turned upside down manually or by a control signal of the control device 66. By bringing the pipette 110 into an upright state in advance, the process can smoothly proceed to both the dilution step and the administration preparation step which are described later.

### <<Dilution step>>

As illustrated in Fig. 3, the dilution step is a step of diluting heparin introduced into the pipette 110 with a priming liquid in the pipette 110 by introducing the priming liquid into the pipette 110. Note that when it is not necessary to dilute heparin, the dilution step is omitted, and the process only needs to proceed to the administration preparation step described later.

### <Dilution step 1>

Fig. 3(a) illustrates a first step of the dilution step.

The processor of the control device 66 outputs a control signal to bring the medicinal liquid clamping means 120 into a closed state (black). The processor of the control device 66 outputs a control signal to drive the blood pump 50, thereby rotating a rotor (not illustrated) of the blood pump 50 forward to introduce the priming liquid into the arterial side blood circuit 30. Note that the forward rotation is a direction in which liquid flows from the arterial side blood circuit 30 to the venous side blood circuit 40. A reverse rotation (opposite rotation) is a direction in which liquid flows from the venous side blood circuit 40 to the arterial side blood circuit 30. For example, a priming liquid containing bag containing the priming liquid is connected to the arterial side blood circuit 30 (not illustrated). The blood pump 50 guides the priming liquid from the priming liquid containing bag to the arterial side blood circuit 30, whereby the priming liquid can be led out to the arterial side blood circuit 30. After a desired amount of the priming liquid is led out to the arterial side blood circuit 30, the blood pump 50 is stopped.

### <Dilution step 2>

Fig. 3(b) illustrates a second step of the dilution step.

The processor of the control device 66 outputs a control signal to maintain the medicinal liquid clamping means 120 in a closed state (black). The processor of the control device 66 outputs a control signal to drive the liquid level adjusting pump 140 to suck the diaphragm 131 of the diaphragm pump 130. Note that, in the dilution step 2, the blood pump 50 is stopped.

### <Dilution step 3>

Fig. 3(c) illustrates a last step of the dilution step.

When the blood pump 50 is operating, the processor of the control device 66 outputs a control signal to stop the blood pump 50 to bring the arterial clamping means 36 into a closed state. The processor of the control device 66 outputs a control signal to bring the medicinal liquid clamping means 120 into an open state (white).

The processor of the control device 66 outputs a control signal to drive the liquid level adjusting pump 140 to press the diaphragm 131 of the diaphragm pump 130. As a result, the inside of the arterial side blood circuit 30 can be brought into a positive pressure state. The inside of the arterial side blood circuit 30 is brought into the positive pressure state, whereby the priming liquid introduced into the arterial side blood circuit 30 is pushed out toward the pipette 110 and introduced into the pipette 110 via the medicinal liquid line 100.

An amount of priming liquid corresponding to a difference between deformation due to pressing of the diaphragm 131 of the diaphragm pump 130 and deformation due to suction of the diaphragm 131 of the diaphragm pump 130 is introduced into the pipette 110. By operating the diaphragm 131 of the diaphragm pump 130 at least once, a desired amount of priming liquid can be introduced from the arterial side blood circuit 30 into the pipette 110.

In this way, heparin contained in the pipette 110 can be diluted with the priming liquid in the pipette 110. Hereinbelow, heparin diluted with a priming liquid is referred to as diluted heparin.

The liquid level detection device 150 detects the amount of diluted heparin stored in the pipette 110. A detection signal indicating the amount of diluted heparin is transmitted to the processor of the control device 66. The processor of the control device 66 can determine whether or not a desired amount of diluted heparin has been stored in the pipette 110. The processor of the control device 66 can calculate a concentration of diluted heparin.

### <<Administration preparation step>>

As illustrated in Fig. 4, the administration preparation step is a step of allowing undiluted heparin or diluted heparin in the pipette 110 to be administered to a patient. Note that, in the administration preparation step, the blood pump 50 is stopped. When heparin is diluted, the process proceeds from the dilution step to the administration preparation step. When it is not necessary to dilute heparin, the process immediately proceeds from the filling step to the administration preparation step.

### <Administration preparation step 1>

Fig. 4(a) illustrates a first step of the administration preparation step.

The processor of the control device 66 outputs a control signal to bring the medicinal liquid clamping means 120 into a closed state (black). The processor of the control device 66 outputs a control signal to drive the liquid level adjusting pump 140 to press the diaphragm 131 of the diaphragm pump 130.

### <Administration preparation step 2>

Fig. 4(b) illustrates a last step of the administration preparation step.

When the blood pump is operating, the processor of the control device 66 outputs a control signal to stop the blood pump to bring the arterial clamping means 36 into a closed state (black). The processor of the control device 66 outputs a control signal to bring the medicinal liquid clamping means 120 into an open state (white). The processor of the control device 66 outputs a control signal to drive the liquid level adjusting pump 140 to suck the diaphragm 131 of the diaphragm pump 130. As a result, the inside of the pipette 110 and the inside of the arterial side blood circuit 30 can be brought into a negative pressure state. The inside of the pipette 110 and the inside of the arterial side blood circuit 30 are brought into the negative pressure state, whereby diluted heparin contained in the pipette 110 is sucked out from the pipette 110 and introduced into the arterial side blood circuit 30 via the medicinal liquid line 100.

An amount of diluted heparin corresponding to a difference between deformation due to pressing of the diaphragm 131 of the diaphragm pump 130 and deformation due to suction of the diaphragm 131 of the diaphragm pump 130 flows through the medicinal liquid line 100 and is led out to the arterial side blood circuit 30. By operating the diaphragm 131 of the diaphragm pump 130 at least once, a desired amount of diluted heparin can be led out to the arterial side blood circuit 30.

As described above, by the filling step, the dilution step, and the administration preparation step described above, heparin can be diluted with the priming liquid in the pipette 110, and a desired amount of diluted heparin can be administered to a patient. Note that when heparin is not diluted with the priming liquid, a desired amount of undiluted heparin can be administered to a patient by the filling step and the administration preparation step described above.

### <<Medicinal liquid supply apparatus 10A according to first embodiment, filling step, dilution step, and administration preparation step>>

According to the medicinal liquid supply apparatus 10A according to the first embodiment, heparin can be introduced into the pipette 110, the priming liquid can be introduced into the pipette 110, and diluted heparin can be led out to the arterial side blood circuit 30, using the diaphragm pump 130 and the liquid level adjusting pump 140 which are disposed in the blood circuit. The diaphragm pump 130 and the liquid level adjusting pump 140 can be effectively utilized in the filling step, the dilution step, and the administration preparation step.

Since the diaphragm pump 130, the liquid level adjusting pump 140, and the like can be controlled by the control device 66 of the dialysis device main body 60, a heparin dilution operation and a heparin administration preparation operation can be automated, reproducibility of a dilution concentration with the priming liquid and the like can be enhanced, and the operation can be performed stably. The automation can simplify operation and reduce a burden on a user. Furthermore, the filling step, the dilution step, and the administration preparation step, or the filling step and the administration preparation step can be continuously performed.

In addition, the pipette 110 has the extending portion 113 thinner than the containing portion 112. Therefore, the extending portion 113 can be easily inserted into or extracted from an opening or the like of a container such as a vial containing heparin. In this way, heparin can be introduced into the pipette 110 with less influence of the shapes and sizes of the container and the opening. Heparin desired by a user can be used. Heparin contained in a container can be used for treatment to be performed a plurality of times. Since it is not necessary to administer heparin by using a syringe, treatment cost can be reduced.

The pipette 110 can be used not only for introduction and storage of heparin but also for introduction and storage of the priming liquid and dilution of heparin, and the pipette 110 can be effectively used and the number of members can be reduced. In addition, by introducing the priming liquid into the pipette 110, heparin in the pipette 110 can be diluted at the same time, and the dilution step can be simplified.

By using the flexible medicinal liquid line 100, the pipette 110 or the like can be disposed at a position away from the blood circuit. It is possible to increase the degree of freedom of disposition of the pipette 110 and the like. The pipette 110 and the like can be disposed so as not to interfere with other devices and the like, and operability of a user can be improved.

Since the medicinal liquid line 100 is shared by both the dilution step of introducing the priming liquid from the arterial side blood circuit 30 into the pipette 110 and the administration preparation step of leading out diluted heparin from the pipette 110 to the arterial side blood circuit 30, the number of members can be reduced, and the configuration can be simplified.

### <<<<Second embodiment>>>>

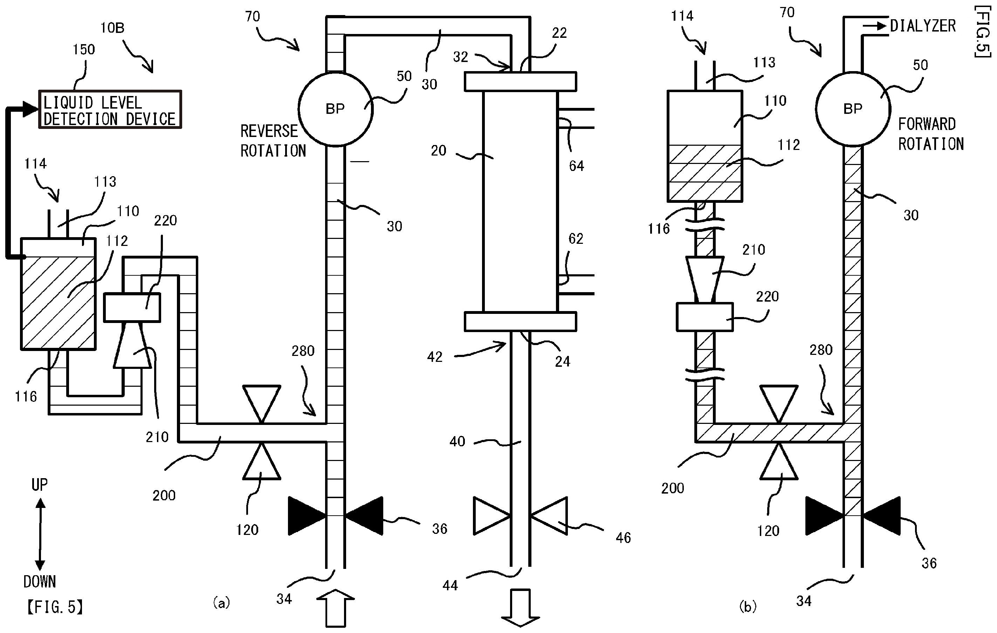

Fig. 5 is a schematic diagram illustrating a filling step in a medicinal liquid supply apparatus 10B according to a second embodiment. Also in the second embodiment, the medicinal liquid supply apparatus 10B is connected to a blood purification apparatus 70 and applied to a hemodialysis apparatus. Note that in the second embodiment, the blood purification apparatus 70 has a similar configuration to that of the first embodiment except that the blood purification apparatus 70 does not include the diaphragm pump 130 and the liquid level adjusting pump 140. In the second embodiment, similar components to those of the first embodiment are denoted by the same reference numerals. In Fig. 5, a dialysis device main body 60 and a control device 66 are not illustrated.

### <<<Configuration of blood purification apparatus 70>>>

The blood purification apparatus 70 of the second embodiment mainly includes:
a dialyzer 20 having a blood purification function;
a blood circuit to which an arterial side blood circuit 30 and a venous side blood circuit 40 are connected;
a blood pump 50;
a dialysis device main body 60 (not illustrated) to which a dialysate introduction line 62 and a dialysate discharge line 64 are connected;
a control device 66 (not illustrated);
a medicinal liquid clamping means 120;
a liquid level detection device 150; and
an intravenous drip sensor 220.

The blood purification apparatus 70 of the second embodiment is different from the blood purification apparatus 70 of the first embodiment in including the intravenous drip sensor 220 and not including the diaphragm pump 130 and the liquid level adjusting pump 140.

### <<<Configuration of medicinal liquid supply apparatus 10B>>>

The medicinal liquid supply apparatus 10B can be disposed outside the blood circuit, and can be used with less change to a conventional blood circuit.

As illustrated in Fig. 5, the medicinal liquid supply apparatus 10B mainly includes:
a medicinal liquid line 200;
a pipette 110; and
a dropping chamber 210.

The pipette 110 has a similar configuration to that of the first embodiment and functions in a similar manner.

### <<Medicinal liquid line 200>>

The medicinal liquid line 200 is connected to the blood circuit. Liquid can flow between the medicinal liquid line 200 and the blood circuit. Examples of the liquid include, but are not limited to, a priming liquid and diluted heparin. For example, the medicinal liquid line 200 is connected to and communicates with the arterial side blood circuit 30. The medicinal liquid line 200 is preferably constituted by a flexible tube or the like. The pipette 110 can be disposed at a position away from the arterial side blood circuit 30.

The medicinal liquid line 200 is connected to the arterial side blood circuit 30 at a connection portion 280. The medicinal liquid line 200 is preferably detachably connected at the connection portion 280. Similarly to the medicinal liquid line 100, the medicinal liquid line 200 can be used as a disposable instrument.

### <Dropping chamber 210>

The dropping chamber 210 separates liquid continuously flowing down into independent droplets and drops the separated droplets by action of gravity. The dropping chamber 210 is a member used for so-called intravenous drip. Specifically, the dropping chamber 210 drops undiluted heparin or diluted heparin in a form of drops. Details will be described later.

### <Intravenous drip sensor 220>

The intravenous drip sensor 220 detects the number of drops (hereinafter, referred to as a number of drops) dropped in the dropping chamber 210 and outputs a detection signal indicating the number of drops to the dialysis device. The control device 66 of the dialysis device main body 60 can execute processing based on the number of drops. Note that the control device 66 of the dialysis device main body 60 has a similar configuration to that of the first embodiment and functions in a similar manner. The control device 66 outputs control signals for controlling various control devices, and detection signals indicating results detected by various detection devices are input to the control device 66.

Note that, in the second embodiment, the intravenous drip sensor 220 is detachably disposed in the dropping chamber 210, but the present invention is not limited thereto. The intravenous drip sensor 220 may be integrated with the dropping chamber 210. The intravenous drip sensor 220 may be any sensor as long as it can detect the number of drops in the dropping chamber 210 and output a detection signal.

### <<Another configuration of medicinal liquid supply apparatus 10B>>

The example has been described in which the blood purification apparatus 70 of the second embodiment mainly includes:
the dialyzer 20 having a blood purification function;
the blood circuit to which the arterial side blood circuit 30 and the venous side blood circuit 40 are connected;
the blood pump 50;
the dialysis device main body 60 to which the dialysate introduction line 62 and the dialysate discharge line 64 are connected;
the control device 66;
the medicinal liquid clamping means 120;
the liquid level detection device 150; and
the intravenous drip sensor 220, and
the medicinal liquid supply apparatus 10B mainly includes:
the medicinal liquid line 200;
the pipette 110; and
the dropping chamber 210.

The present invention is not limited to this configuration, and the medicinal liquid supply apparatus 10B may include at least one of the medicinal liquid clamping means 120, the liquid level detection device 150, and the intravenous drip sensor 220. For example, a control device (not illustrated) including a processor (processing device) or the like is disposed in the medicinal liquid supply apparatus 10B to make it possible to output a control signal from the control device or to input a detection signal to the control device. Furthermore, the control device of the medicinal liquid supply apparatus 10B is communicably connected to the control device 66 of the blood purification apparatus 70. Control is performed by appropriately dividing the medicinal liquid clamping means 120, the liquid level detection device 150, and the intravenous drip sensor 220 into those controlled by the control device 66 of the blood purification apparatus 70 and those controlled by the control device of the medicinal liquid supply apparatus 10B.

Note that only the control device of the medicinal liquid supply apparatus 10B may control all of the medicinal liquid clamping means 120, the liquid level detection device 150, and the intravenous drip sensor 220. The medicinal liquid clamping means 120 is controlled by a control signal output from the control device of the medicinal liquid supply apparatus 10B, and detection signals output from the liquid level detection device 150 and the intravenous drip sensor 220 are input to the control device of the medicinal liquid supply apparatus 10B.

### <<Operation performed by medicinal liquid supply apparatus 10B>>

When heparin is diluted with the priming liquid, an operationperformed by the medicinal liquid supply apparatus 10B also includes a filling step, a dilution step, and an administration preparation step. When heparin is not diluted with the priming liquid, the operationperformed by the medicinal liquid supply apparatus 10B includes the filling step and the administration preparation step. Note that Fig. 5 illustrates the dilution step (a) and the administration preparation step (b).

In Fig. 5, a valve indicated in white indicates an open state, and a valve indicated in black indicates a closed state. In Fig. 5, presence of heparin is indicated by oblique lines, and presence of a priming liquid is indicated by horizontal lines.

### <<Filling step>>

In the second embodiment, unlike the first embodiment, heparin is introduced into the pipette 110 by an operator's operation (manual operation) (see Fig. 2).

As illustrated in Fig. 5(a), the dilution step is a step of diluting heparin introduced into the pipette 110 with a priming liquid in the pipette 110 by introducing the priming liquid into the pipette 110. Note that when it is not necessary to dilute heparin, the dilution step is omitted, and the process only needs to immediately proceed to the administration preparation step described later.

In the second embodiment, unlike the first embodiment, the priming liquid is introduced from the arterial side blood circuit 30 to the pipette 110 by reversely rotating the blood pump 50. The blood pump 50 can be reversely rotated by a control signal from the control device 66. The liquid level detection device 150 can determine whether the priming liquid in the pipette 110 has reached a required amount.

The dropping chamber 210 is disposed by being turned upside down. The dropping chamber 210 may be turned upside down manually or by a control signal of the control device 66. By disposing a drive device (not illustrated) such as a motor in the dropping chamber 210, the pipette 110 can be turned upside down by a control signal output from the control device 66.

An operation of turning the dropping chamber 210 upside down may be performed manually or by using a drive device (not illustrated) such as a motor. The control device 66 of the blood purification apparatus 70 outputs a control signal for turning the dropping chamber 210 upside down to the drive device such as a motor. When the control device 66 of the blood purification apparatus 70 outputs an upright command signal, the dropping chamber 210 is brought into an upright state by the drive device such as a motor. The control device 66 can determine that the state of the dropping chamber 210 is the upright state by outputting the upright command signal. When the control device 66 of the blood purification apparatus 70 outputs an inverted command signal, the dropping chamber 210 is brought into an inverted state by the drive device such as a motor. The control device 66 can determine that the state of the dropping chamber 210 is the inverted state by outputting the inverted command signal.

In addition, a sensor (not illustrated) for detecting whether the dropping chamber 210 is in the upright state or the inverted state may be disposed. The sensor may be any sensor as long as it detects the upright state or the inverted state of the dropping chamber 210 and outputs a signal indicating a detection result, regardless of whether the sensor is an optical sensor, a mechanical sensor, or a sensor using another detection method. The control device 66 of the blood purification apparatus 70 can acquire the state of the dropping chamber 210 by receiving a signal indicating a detection result, emitted from the sensor. Even when a failure occurs in the drive device such as a motor, the state of the dropping chamber 210 can be accurately acquired.

Furthermore, when an operator manually turns the dropping chamber 210 upside down, the operator operates a touch panel after an operation of turning the dropping chamber 210 upside down is completed. For example, when the operator brings the dropping chamber 210 into the upright state, the operator inputs, to the touch panel, information indicating that the dropping chamber 210 has been brought into the upright state. By this operation, the touch panel outputs a signal indicating that the dropping chamber 210 has been brought into the upright state to the control device. As a result, the control device 66 can determine that the state of the dropping chamber 210 is the upright state. On the other hand, when the operator brings the dropping chamber 210 into the inverted state, the operator inputs, from the touch panel, information indicating that the dropping chamber 210 has been brought into the inverted state. By this operation, the touch panel outputs a signal indicating that the dropping chamber 210 has been brought into the inverted state to the control device. As a result, the control device 66 can determine that the state of the dropping chamber 210 is the inverted state.

Note that the pipette 110 only needs to be disposed so as to be located above the dropping chamber 210 all the time in both the upright state in which the dropping chamber 210 is turned upside down and the inverted state.

When the blood pump 50 is operating, the processor of the control device 66 outputs a control signal to stop the blood pump 50 to bring the arterial clamping means 36 into a closed state (black). The processor of the control device 66 outputs a control signal to bring the medicinal liquid clamping means 120 into an open state (white).

The processor of the control device 66 outputs a control signal to drive the blood pump 50. By driving of the blood pump 50, the priming liquid introduced into the arterial side blood circuit 30 is pushed out, flows through the medicinal liquid line 200, and is introduced into the pipette 110 via the medicinal liquid clamping means 120 and the dropping chamber 210. In this way, the priming liquid can be introduced into the pipette 110.

An amount of the priming liquid corresponding to the amount for driving the blood pump 50 is introduced into the pipette 110. In this way, heparin introduced into the pipette 110 can be diluted with the priming liquid in the pipette 110. Also in the second embodiment, similarly to the first embodiment, heparin diluted with the priming liquid is referred to as diluted heparin.

The liquid level detection device 150 detects the amount of diluted heparin stored in the pipette 110. A detection signal indicating the amount of diluted heparin is transmitted to the processor of the control device 66. The processor of the control device 66 can determine whether or not a desired amount of undiluted heparin or diluted heparin has been stored in the pipette 110. The processor of the control device 66 can calculate a concentration of diluted heparin.

### <<Administration preparation step>>

As illustrated in Fig. 5(b), the administration preparation step is a step of allowing undiluted heparin or diluted heparin in the pipette 110 to be administered to a patient.

The dropping chamber 210 is turned upside down to be returned to an original state. The dropping chamber 210 may be turned upside down manually or by a control signal of the control device 66.

The processor of the control device 66 outputs a control signal to drive the blood pump 50. By forward rotating a rotor (not illustrated) of the blood pump 50, diluted heparin corresponding to the amount of the blood pump 50 driven flows through the medicinal liquid line 200 and is led out to the arterial side blood circuit 30.

The intravenous drip sensor 220 detects dropwise heparin every time undiluted heparin or diluted heparin is dropped in the dropping chamber 210. The intravenous drip sensor 220 outputs a detection signal indicating that heparin has been dropped to the control device 66. The control device 66 can count the number of drops of undiluted heparin or diluted heparin and calculate the total amount of the administered undiluted heparin or diluted heparin by receiving the detection signal. The control device 66 executes processing based on the total amount of undiluted heparin or diluted heparin. Note that the volume of one drop of undiluted heparin or diluted heparin is measured in advance and stored in the RAM of the control device 66 or the like.

### <<Medicinal liquid supply apparatus 10B according to second embodiment, filling step, dilution step, and administration preparation step>>

According to the medicinal liquid supply apparatus 10B according to the second embodiment, the priming liquid can be introduced into the pipette 110, and diluted heparin can be led out to the arterial side blood circuit 30, using the blood pump 50 disposed in the blood circuit. The blood pump 50 can be effectively utilized in the dilution step and the administration preparation step.

According to the medicinal liquid supply apparatus 10B according to the second embodiment, heparin can be diluted in the pipette 110, and undiluted heparin or diluted heparin can be administered with a simple configuration without using the diaphragm pump 130 and the liquid level adjusting pump 140 of the first embodiment. The dilution step and the administration preparation step can be performed without being limited to the configuration of the blood circuit.

According to the medicinal liquid supply apparatus 10B according to the second embodiment, it is possible to measure undiluted heparin or diluted heparin in the administration preparation step using the dropping chamber 210 and the intravenous drip sensor 220. If the medicinal liquid supply apparatus 10B can cooperate with the dialysis device main body 60, the medicinal liquid supply apparatus 10B can be configured by utilizing members used at the site of dialysis treatment. That is, at the site of dialysis treatment, the control device 66 of the blood purification apparatus 70 receives a detection signal output from the intravenous drip sensor 220, and a device, a component, a member, and the like which control the blood pump 50 are used based on the number of drops. The medicinal liquid supply apparatus 10B according to the second embodiment can be configured by effectively utilizing these devices, components, members, and the like.

The pipette 110 can be used not only for introduction and storage of heparin but also for introduction and storage of the priming liquid and dilution of heparin, and the pipette 110 can be effectively used and the number of members can be reduced. In addition, by introducing the priming liquid into the pipette 110, heparin in the pipette 110 can be diluted at the same time, and the dilution step can be simplified.

By using the flexible medicinal liquid line 200, the pipette 110 or the like can be disposed at a position away from the blood circuit. It is possible to increase the degree of freedom of disposition of the pipette 110 and the like The pipette 110 and the like can be disposed so as not to interfere with other devices and the like, and operability of a user can be improved.

Since the medicinal liquid line 200 is shared by both the dilution step of introducing a priming liquid from the arterial side blood circuit 30 to the pipette 110 and the administration preparation step of leading out diluted heparin from the pipette 110 to the arterial side blood circuit 30, the number of members can be reduced, and the configuration can be simplified.

### <<<<Third embodiment>>>>

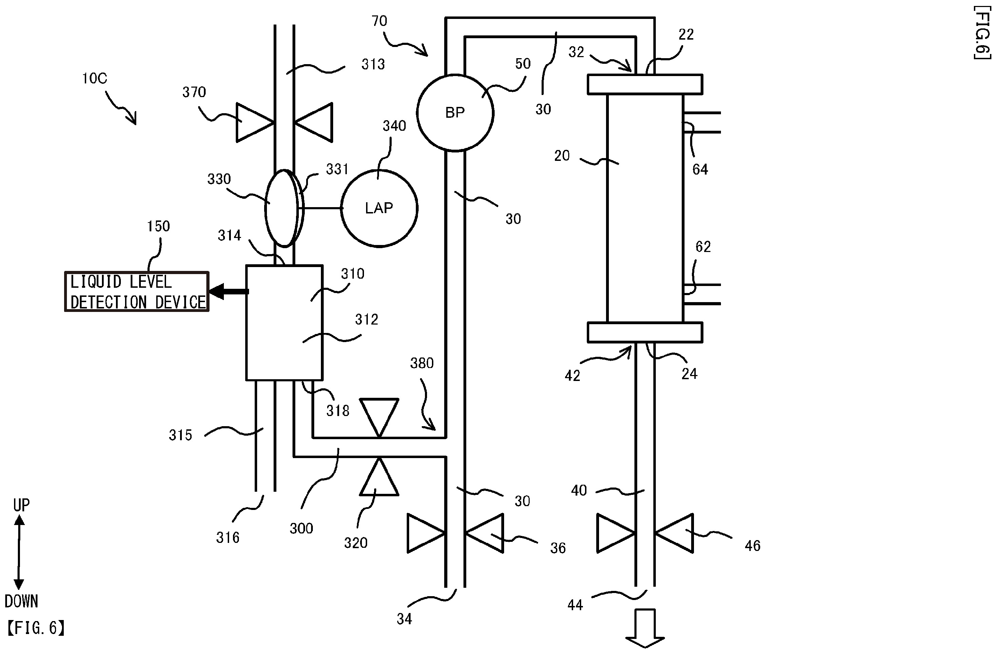

Fig. 6 is a schematic diagram illustrating a configuration of a medicinal liquid supply apparatus 10C according to a third embodiment. Similarly to the medicinal liquid supply apparatus 10A of the first embodiment, the medicinal liquid supply apparatus 10C of the third embodiment is connected to a blood purification apparatus 70 and applied to a hemodialysis apparatus. Also in the third embodiment, similar components to those of the first embodiment are denoted by the same reference numerals.

In the third embodiment, the blood purification apparatus 70 mainly includes:
a dialyzer 20 having a blood purification function;
a blood circuit to which an arterial side blood circuit 30 and a venous side blood circuit 40 are connected;
a blood pump 50;
a dialysis device main body 60 (not illustrated) to which a dialysate introduction line 62 and a dialysate discharge line 64 are connected;
a control device 66 (not illustrated);
a diaphragm pump 330;
a liquid level adjusting pump 340;
a solenoid valve 370;
an extending portion 313;
a liquid level detection device 150; and
a medicinal liquid clamping means 320.

Note that, in Fig. 6, a dialysis device main body 60 and a control device 66 are not illustrated. Note that the control device 66 of the dialysis device main body 60 has a similar configuration to those of the first and second embodiments and functions in a similar manner. The control device 66 outputs control signals for controlling various control devices, and detection signals indicating results detected by various detection devices are input to the control device 66.

### <Diaphragm pump 330>

The diaphragm pump 330 has a deformable diaphragm 331. The diaphragm pump 330 controls a flow of liquid by deformation of the diaphragm 331. The diaphragm 331 of the diaphragm pump 330 is driven by the liquid level adjusting pump 340. The diaphragm pump 330 presses or sucks the diaphragm 331 by the liquid level adjusting pump 340. By deforming the diaphragm 331 by pressing or suction of the diaphragm 331 to generate a negative pressure in the arterial side blood circuit 30 or the venous side blood circuit 40, a flow of liquid in the arterial side blood circuit 30 or the venous side blood circuit 40 is controlled. The diaphragm pump 330 can repeat pressing and suction by the liquid level adjusting pump 340.

Note that since the diaphragm pump 330 is not in contact with blood, the diaphragm pump 330 can be reused as a component of the blood purification apparatus 70 a plurality of times.

### <Liquid level adjusting pump 340>

The liquid level adjusting pump 340 deforms the diaphragm 331 of the diaphragm pump 330. The liquid level adjusting pump 340 presses or sucks the diaphragm 331 of the diaphragm pump 330.

The liquid level adjusting pump 340 includes a drive device (not illustrated) such as a solenoid or a motor. In the liquid level adjusting pump 340, the drive device is driven by a control signal output from the control device 66, whereby the liquid level adjusting pump 340 performs pressing or suction.

### <Solenoid valve 370>

The solenoid valve 370 is actuated by a solenoid (not illustrated). The control device 66 outputs a control signal for controlling the solenoid. The solenoid is controlled by a control signal output from the control device 66, and the solenoid valve 370 is brought into an opened state or a closed state. When the solenoid valve 370 is in an open state (indicated in white), the pipette 310 communicates with the outside, and air in the pipette 310 can be discharged to the outside in response to driving of the diaphragm pump 330. When the solenoid valve 370 is in a closed state (indicated in black), the pipette 310 does not communicate with the outside, and even if the diaphragm pump 330 is driven, air cannot flow between the pipette 310 and the outside.

### <Extending portion 313>

The extending portion 313 has a long and substantially cylindrical shape. The extending portion 313 extends in a direction away from a containing portion 312 above the containing portion 312. The extending portion 313 has a smaller diameter than the containing portion 312. The extending portion 313 is disposed concentrically with the containing portion 312. That is, the extending portion 313 is disposed such that a central axis of the extending portion 313 coincides with a central axis of the containing portion 312. The extending portion 313 communicates with the outside.

The diaphragm pump 330 and the solenoid valve 370 are connected to the extending portion 313. By controlling the diaphragm pump 330 and the solenoid valve 370, air in the containing portion 312 can be discharged to the outside, and heparin and/or a priming liquid can be introduced into the pipette 310. Details will be described later.

### <<<Configuration of medicinal liquid supply apparatus 10C>>>

The medicinal liquid supply apparatus 10C can be disposed outside the blood circuit, and can be used with less change to a conventional blood circuit.

As illustrated in Fig. 6, the medicinal liquid supply apparatus 10C mainly includes;
a medicinal liquid line 300; and
the pipette 310.

### <<Medicinal liquid line 300>>

The medicinal liquid line 300 is connected to the blood circuit. Liquid can flow between the medicinal liquid line 300 and the blood circuit. Examples of the liquid include, but are not limited to, a priming liquid and diluted heparin. For example, the medicinal liquid line 300 is connected to and communicates with the arterial side blood circuit 30. The medicinal liquid line 300 is preferably constituted by a flexible tube or the like. The pipette 310 can be disposed at a position away from the arterial side blood circuit 30.

The medicinal liquid line 300 is connected to the arterial side blood circuit 30 at a connection portion 380. The medicinal liquid line 300 is preferably detachably connected at the connection portion 380. Similarly to the medicinal liquid lines 100 and 200, the medicinal liquid line 300 can be used as a disposable instrument.

### <<Pipette 310>>

As in the first and second embodiments, heparin and/or a priming liquid is introduced into the pipette 310. Also in the third embodiment, heparin is diluted with the priming liquid in the pipette 310.

The pipette 310 has an overall long shape. The pipette 310 is disposed such that a longitudinal direction thereof is along the up-down direction. Unlike the first embodiment, the pipette 310 does not need to be turned upside down.

The pipette 310 mainly has the containing portion 312, an upper end opening 314, a first lower end opening 316, and a second lower end opening 318.

### <Containing portion 312>

The containing portion 312 has a long and substantially cylindrical shape. Heparin and/or a priming liquid is introduced into the containing portion 312. In the containing portion 312, heparin is diluted with the priming liquid. When the priming liquid is not introduced into the containing portion 312, heparin is not diluted with the priming liquid in the containing portion 312, and heparin is simply contained therein.

### <Upper end opening 314>

The pipette 310 has the upper end opening 314 in an upper portion thereof. The upper end opening 314 is connected to the extending portion 313. The extending portion 313 communicates with the outside, and can discharge air from the containing portion 312 to the outside via the upper end opening 314 and the extending portion 313.

### <Extending portion 315>

An extending portion 315 has a long and substantially cylindrical shape. The extending portion 315 extends in a direction (downward direction) away from the containing portion 312 below the containing portion 312. The extending portion 315 has a smaller diameter than the containing portion 312. By making the extending portion 315 thinner than the containing portion 312, the extending portion 315 can be inserted into and extracted from openings of various containers, and heparin contained in containers having various shapes and sizes can be introduced into the containing portion 312. In particular, the extending portion 315 is preferably formed to be thinner (tapered) as being away from the containing portion 312. The extending portion 315 can be more easily inserted into and extracted from openings of various containers.

### <First lower end opening 316>

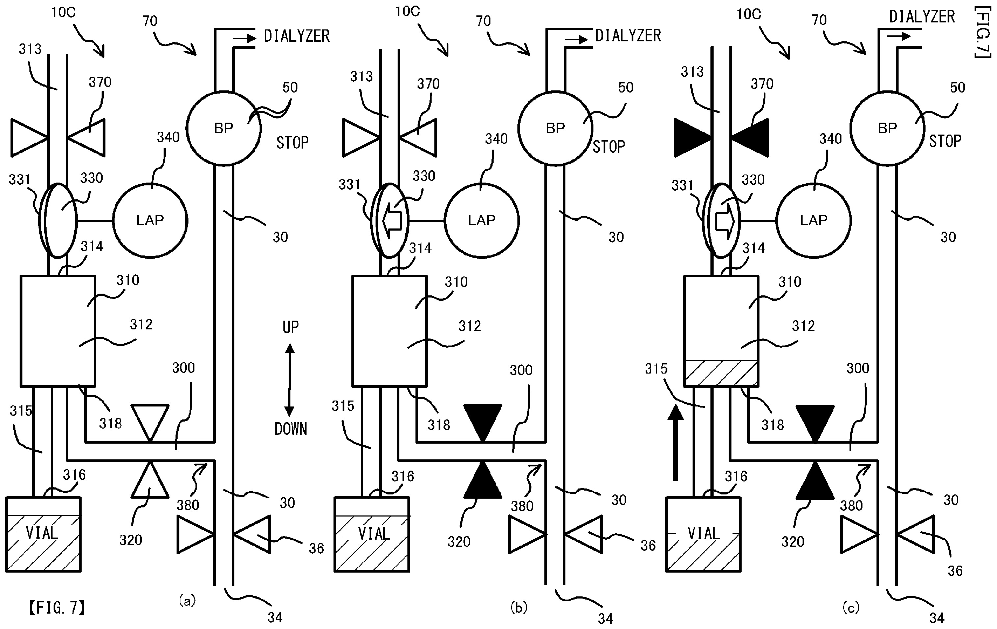

The extending portion 315 has the first lower end opening 316 at a lower end thereof (a portion farthest from the containing portion 312). The first lower end opening 316 communicates with the containing portion 312 and the extending portion 315. The first lower end opening 316 can be connected to a vial (see Fig. 7). Heparin stored in the vial is introduced into the containing portion 312 from the first lower end opening 316 via the extending portion 315.

### <Second lower end opening 318>

The pipette 310 has the second lower end opening 318 in a lower portion thereof. The second lower end opening 318 communicates with the containing portion 312. The pipette 310 is connected to the medicinal liquid line 300 at the second lower end opening 318. A priming liquid is introduced from the second lower end opening 318 into the containing portion 312 via the medicinal liquid line 300. In addition, heparin diluted with the priming liquid is led out from the containing portion 312 to the medicinal liquid line 300 via the second lower end opening 318. When heparin is not diluted with the priming liquid, the priming liquid is not introduced into the containing portion 312, and undiluted heparin is led out from the containing portion 312 to the medicinal liquid line 300 via the second lower end opening 318.

### <Medicinal liquid clamping means 320>

The medicinal liquid clamping means 320 is disposed in the medicinal liquid line 300. The medicinal liquid clamping means 320 is in either an open state or a closed state. When the medicinal liquid clamping means 320 is in an open state, liquid can flow in the medicinal liquid line 300. When the medicinal liquid clamping means 320 is in a closed state, liquid cannot flow in the medicinal liquid line 300.

The medicinal liquid clamping means 320 includes a drive device (not illustrated) such as a solenoid or a motor. In the medicinal liquid clamping means 320, the drive device is driven by a control signal output from the control device 66, whereby the medicinal liquid clamping means 320 is brought into an open state or a closed state.

### <<Another configuration of medicinal liquid supply apparatus 10C>>

In the third embodiment, the example has been described in which the blood purification apparatus 70 mainly includes:
the dialyzer 20 having a blood purification function;
the blood circuit to which the arterial side blood circuit 30 and the venous side blood circuit 40 are connected;
the blood pump 50;
the dialysis device main body 60 to which the dialysate introduction line 62 and the dialysate discharge line 64 are connected;
the control device 66;
the diaphragm pump 330;
the liquid level adjusting pump 340;
the solenoid valve 370;
the extending portion 313; and
the liquid level detection device 150, and
the medicinal liquid supply apparatus 10C mainly includes:
   the medicinal liquid line 300; and
   the pipette 310.

The present invention is not limited to this configuration, and the medicinal liquid supply apparatus 10C may include at least one of the diaphragm pump 330, the liquid level adjusting pump 340, the solenoid valve 370, the extending portion 313, the liquid level detection device 150, and the medicinal liquid clamping means 320. For example, a control device (not illustrated) including a processor (processing device) or the like is disposed in the medicinal liquid supply apparatus 10C to make it possible to output a control signal from the control device or to input a detection signal to the control device. Furthermore, the control device of the medicinal liquid supply apparatus 10C is communicably connected to the control device 66 of the blood purification apparatus 70. Control is performed by appropriately dividing the diaphragm pump 330, the liquid level adjusting pump 340, the solenoid valve 370, and the medicinal liquid clamping means 320 into those controlled by the control device 66 of the blood purification apparatus 70 and those controlled by the control device of the medicinal liquid supply apparatus 10C.

Note that only the control device of the medicinal liquid supply apparatus 10C may control all of the diaphragm pump 330, the liquid level adjusting pump 340, the solenoid valve 370, and the medicinal liquid clamping means 320. The diaphragm pump 330, the liquid level adjusting pump 340, the solenoid valve 370, and the medicinal liquid clamping means 320 are controlled by a control signal output from the control device of the medicinal liquid supply apparatus 10C, and a detection signal output from the liquid level detection device 150 is input to the control device of the medicinal liquid supply apparatus 10C.

### <<Operation performed by medicinal liquid supply apparatus 10C>>

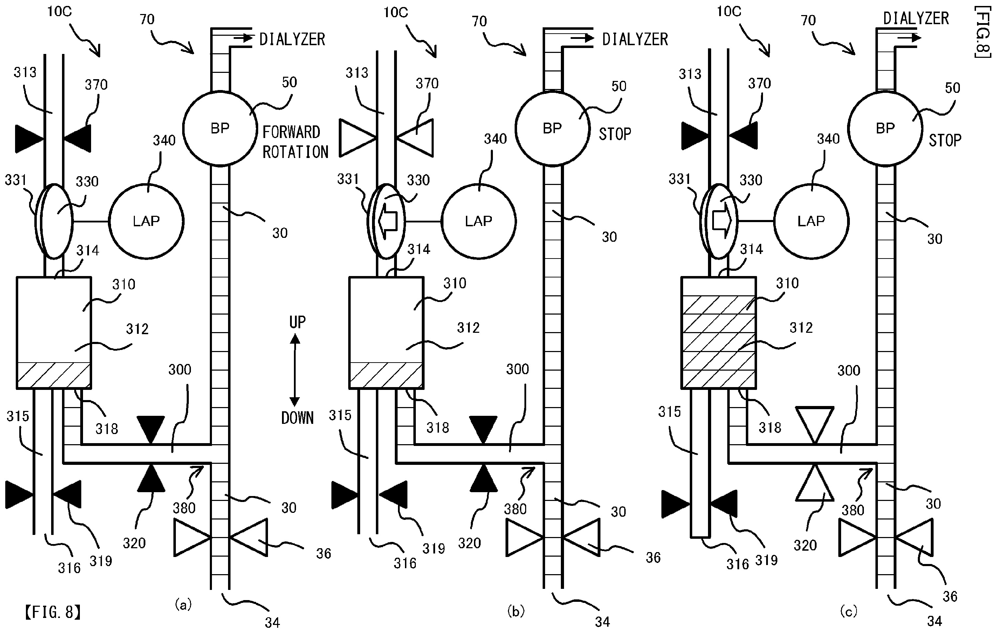

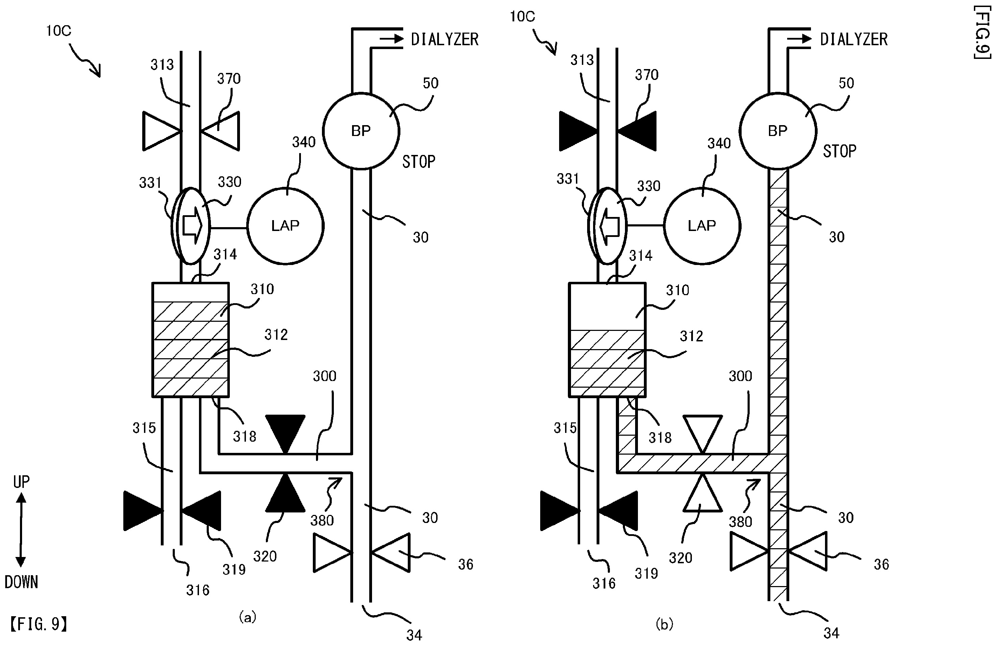

Fig. 7 is a schematic diagram illustrating a filling step in the medicinal liquid supply apparatus 10C according to the third embodiment. Fig. 8 is a schematic diagram illustrating a dilution step in the medicinal liquid supply apparatus 10C according to the third embodiment. Fig. 9 is a schematic diagram illustrating an administration preparation step in the medicinal liquid supply apparatus 10C according to the third embodiment. As illustrated in Figs. 7 to 9, when heparin is diluted with the priming liquid, an operation performed by the medicinal liquid supply apparatus 10C includes the filling step, the dilution step, and the administration preparation step. When heparin is not diluted with the priming liquid, the operationperformed by the medicinal liquid supply apparatus 10C includes the filling step and the administration preparation step.

Also in Figs. 7 to 9, a valve indicated in white indicates an open state, and a valve indicated in black indicates a closed state. In Figs. 7 to 9, presence of heparin is indicated by oblique lines, and presence of a priming liquid is indicated by horizontal lines. A leftward white arrow on the diaphragm pump 330 indicates that the diaphragm 331 has been pressed, and a rightward white arrow on the diaphragm pump 330 indicates that the diaphragm 331 has been sucked.

### <<Filling step>>

As illustrated in Fig. 7, the filling step is a step of introducing heparin from a vial into the pipette 310. By the filling step, heparin contained in a container such as a vial (such as a container at the time of delivery) can be introduced into the pipette 310. Heparin can be used without depending on the shape of the container or the form of the container, such as an opening of the container, at the time of delivery. It is compatible with a wide variety of containers in which heparin is contained. Note that, in the filling step, the blood pump 50 is stopped.

### <Filling step 1>

Fig. 7(a) illustrates a first step of the filling step. At this time, all of the medicinal liquid clamping means 320, the solenoid valve 370, and the arterial clamping means 36 are in an open state (white). Note that, in the medicinal liquid supply apparatus 10C, the arterial clamping means 36 is in an open state (white) not only in the filling step but also in the dilution step and the administration preparation step. The medicinal liquid supply apparatus 10C does not have to include the arterial clamping means 36. When the medicinal liquid supply apparatus 10C does not include the arterial clamping means 36, control on the arterial clamping means 36 can be omitted. In addition, as illustrated in Figs. 7 to 9, when the medicinal liquid supply apparatus 10C uses a system including the arterial clamping means 36, it is only required to perform control to keep the arterial clamping means 36 in an open state all the time.

First, a vial containing heparin is disposed below the pipette 310. Next, the first lower end opening 316 of the pipette 310 is inserted into the vial to communicate with the vial.

### <Filling step 2>

Fig. 7(b) illustrates a second step of the filling step.

The processor of the control device 66 outputs a control signal to bring the medicinal liquid clamping means 320 into a closed state (black). The processor of the control device 66 outputs a control signal to bring the solenoid valve 370 into an open state (white). The processor of the control device 66 outputs a control signal to drive the liquid level adjusting pump 140 to press the diaphragm 331 of the diaphragm pump 330.

### <Filling step 3>

Fig. 7(c) illustrates a last step of the filling step.

The processor of the control device 66 outputs a control signal to bring the medicinal liquid clamping means 320 into a closed state (black). The processor of the control device 66 outputs a control signal to bring the solenoid valve 370 into a closed state (black). The processor of the control device 66 outputs a control signal to drive the liquid level adjusting pump 140 to suck the diaphragm 331 of the diaphragm pump 330. As a result, the inside of the pipette 310 can be brought into a negative pressure state. The inside of the pipette 310 is brought into the negative pressure state, whereby heparin contained in the vial is sucked from the vial and introduced into the pipette 310.

An amount of heparin corresponding to a difference between deformation due to pressing of the diaphragm 331 of the diaphragm pump 330 and deformation due to suction of the diaphragm 331 of the diaphragm pump 330 is introduced into the pipette 310. By deforming the diaphragm 331 of the diaphragm pump 330 at least once, a desired amount of heparin can be introduced from the vial into the pipette 310.

By the filling step illustrated in Figs. 7(a) to 7(c), heparin can be introduced into the pipette 110 without depending on the shape of a container containing heparin or the form of the container, such as an opening.

As illustrated in Fig. 8, the dilution step is a step of diluting heparin introduced into the pipette 310 with a priming liquid in the pipette 310 by introducing the priming liquid into the pipette 310. Note that, in the dilution step, the blood pump 50 is stopped. When it is not necessary to dilute heparin, the dilution step is omitted, and the process only needs to proceed to the administration preparation step described later.

Fig. 8(a) illustrates a first step of the dilution step.

The processor of the control device 66 outputs a control signal to bring the medicinal liquid clamping means 320 into a closed state (black). The processor of the control device 66 outputs a control signal to rotate the blood pump 50 forward to introduce the priming liquid into the arterial side blood circuit 30. For example, a priming liquid containing bag containing the priming liquid is connected to the arterial side blood circuit 30 (not illustrated). The blood pump 50 guides the priming liquid from the priming liquid containing bag to the arterial side blood circuit 30, whereby the priming liquid can be introduced into the arterial side blood circuit 30.

The pipette 310 is extracted from the vial and the first lower end opening 316 of the pipette 310 is sealed with a clamp 319. By sealing, heparin introduced into pipette 310 can be prevented from leaking from the first lower end opening 316.

Note that a drive device such as a motor may be disposed in the pipette 310, and control may be performed such that the pipette 310 is extracted from the vial and the first lower end opening 316 of the pipette 310 is clamped by a control signal emitted from the control device 66.

### <Dilution step 2>

Fig. 8(b) illustrates a second step of the dilution step.

The processor of the control device 66 outputs a control signal to bring the medicinal liquid clamping means 320 into a closed state (black). The processor of the control device 66 outputs a control signal to bring the solenoid valve 370 into an open state (white). The processor of the control device 66 outputs a control signal to drive the liquid level adjusting pump 140 to press the diaphragm 331 of the diaphragm pump 330.

### <Dilution step 3>

Fig. 8(c) illustrates a last step of the dilution step.

The processor of the control device 66 outputs a control signal to bring the medicinal liquid clamping means 320 into an open state (white). The processor of the control device 66 outputs a control signal to bring the solenoid valve 370 into a closed state (black). The processor of the control device 66 outputs a control signal to drive the liquid level adjusting pump 140 to suck the diaphragm 331 of the diaphragm pump 330. As a result, the inside of the pipette 310 can be brought into a negative pressure state. The inside of the pipette 310 is brought into the negative pressure state, whereby the priming liquid introduced into the arterial side blood circuit 30 is sucked out and introduced into the pipette 310 via the medicinal liquid line 300.

An amount of priming liquid corresponding to a difference between deformation due to pressing of the diaphragm 331 of the diaphragm pump 330 and deformation due to suction of the diaphragm 331 of the diaphragm pump 330 is introduced into the pipette 310. By deforming the diaphragm 331 of the diaphragm pump 330 at least once, a desired amount of priming liquid can be introduced from the arterial side blood circuit 30 into the pipette 310. By introducing the priming liquid into the pipette 310, heparin contained in the pipette 310 can be diluted with the priming liquid in the pipette 310.

Also in the third embodiment, heparin diluted with the priming liquid is referred to as diluted heparin. Similarly to the first embodiment, by disposing the liquid level detection device 150 in the pipette 310, the processor of the control device 66 can determine whether or not a desired amount of undiluted heparin or diluted heparin has been stored in the pipette 310. The processor of the control device 66 can calculate a concentration of heparin.

### <<Administration preparation step>>

As illustrated in Fig. 9, the administration preparation step is a step of allowing undiluted heparin or diluted heparin in the pipette 310 to be administered to a patient. When heparin is diluted, the process proceeds from the dilution step to the administration preparation step. When it is not necessary to dilute heparin, the process immediately proceeds from the filling step to the administration preparation step.

### <Administration preparation step 1>

Fig. 9(a) illustrates a first step of the administration preparation step. Note that, in the administration preparation step 1, the blood pump 50 is stopped.

The processor of the control device 66 outputs a control signal to bring the medicinal liquid clamping means 320 into a closed state (black). The processor of the control device 66 outputs a control signal to bring the solenoid valve 370 into an open state (white). The processor of the control device 66 outputs a control signal to drive the liquid level adjusting pump 340 to suck the diaphragm 331 of the diaphragm pump 330.

### <Administration preparation step 2>

Fig. 9(b) illustrates a last step of the administration preparation step.

When the blood pump is operating, the processor of the control device 66 outputs a control signal to stop the blood pump 50 to bring the medicinal liquid clamping means 320 into an open state (white). The processor of the control device 66 outputs a control signal to bring the solenoid valve 370 into a closed state (black). The processor of the control device 66 outputs a control signal to drive the liquid level adjusting pump 340 to press the diaphragm 331 of the diaphragm pump 330.

Undiluted heparin or diluted heparin in an amount corresponding to deformation of the diaphragm 331 of the diaphragm pump 330 is introduced into the arterial side blood circuit 30. By operating the diaphragm 331 of the diaphragm pump 330 at least once, a desired amount of undiluted heparin or diluted heparin can be introduced into the arterial side blood circuit 30. As a result, the inside of the pipette 310 can be brought into a positive pressure state. The inside of the pipette 310 is brought into the positive pressure state, whereby the undiluted heparin or diluted heparin introduced into the pipette 310 is introduced into the arterial side blood circuit 30 via the medicinal liquid line 300.

As described above, heparin is diluted with the priming liquid in the pipette 310, and undiluted heparin or diluted heparin can be administered to a patient.

### <<Medicinal liquid supply apparatus 10C according to third embodiment, filling step, dilution step, and administration preparation step>>

According to the medicinal liquid supply apparatus 10C according to the third embodiment, heparin can be introduced into the pipette 310, the priming liquid can be introduced into the pipette 310, and diluted heparin can be led out to the arterial side blood circuit 30, using the diaphragm pump 330 and the liquid level adjusting pump 340. The diaphragm pump 330 and the liquid level adjusting pump 340 are disposed separately from the configuration of the blood circuit. Therefore, the filling step, the dilution step, and the administration preparation step can be performed without being limited to the configuration of the blood circuit.

Since the diaphragm pump 330, the liquid level adjusting pump 340, and the like can be controlled by the control device 66 of the dialysis device main body 60, a heparin dilution operation and a heparin administration operation can be automated, reproducibility of a dilution concentration by the priming liquid and the like can be enhanced, and the operation can be performed stably. The automation can simplify operation and reduce a burden on a user. Furthermore, the filling step, the dilution step, and the administration preparation step can be continuously performed.

The pipette 310 has the extending portion 315 extending in a direction away from the containing portion 312. Therefore, the pipette 310 can be connected to the vial without being turned upside down, and heparin can be introduced into the containing portion 312. As described above, since the pipette 310 is not turned upside down, the configuration can be simplified.

In addition, the pipette 310 has the extending portion 315 thinner than the containing portion 312. Therefore, the extending portion 315 can be easily inserted into or extracted from an opening of a container such as a vial containing heparin. In this way, heparin can be introduced into the pipette 310 with less influence of the shapes and sizes of the container and the opening. Heparin desired by a user can be used. Heparin contained in a container can be used for treatment to be performed a plurality of times. Since it is not necessary to administer heparin by using a syringe, treatment cost can be reduced.

The pipette 310 can be used not only for introduction and storage of heparin but also for introduction and storage of the priming liquid and dilution of heparin, and the pipette 310 can be effectively used and the number of members can be reduced. In addition, by introducing the priming liquid into the pipette 310, heparin in the pipette 310 can be diluted at the same time, and the dilution step can be simplified.

By using the flexible medicinal liquid line 300, the pipette 310 or the like can be disposed at a position away from the blood circuit. It is possible to increase the degree of freedom of disposition of the pipette 310 and the like. The pipette 310 and the like can be disposed so as not to interfere with other devices and the like, and operability of a user can be improved.

Since the medicinal liquid line 300 is shared by both the dilution step of introducing a priming liquid from the arterial side blood circuit 30 to the pipette 310 and the administration preparation step of leading out diluted heparin from the pipette 310 to the arterial side blood circuit 30, the number of members can be reduced, and the configuration can be simplified.

### <<<Modifications>>>

In the third embodiment described above, the example has been described in which the blood purification apparatus 70 includes the diaphragm pump 330, the liquid level adjusting pump 340, the solenoid valve 370, and the extending portion 313. However, the medicinal liquid supply apparatus 10C may include the diaphragm pump 330 and the extending portion 313. The diaphragm pump 330 and the liquid level adjusting pump 340 can be controlled not by the control device 66 of the blood purification apparatus 70 but by a control device (not illustrated) of the medicinal liquid supply apparatus 10C.

The control device of the medicinal liquid supply apparatus 10C mainly includes a processor (such as a central processing unit (CPU)), a read only memory (ROM), a random access memory (RAM), an input/output interface (I/O), an interface device (I/F), an auxiliary storage device (such as a hard disk drive (HDD) or a solid state drive (SSD)), an input operation device (such as a touch panel or a keyboard), and the like. The ROM stores programs, constants, and the like for executing various types of processing such as control processing. The RAM temporarily stores values of variables and the like used when a program is executed.

The control device of the medicinal liquid supply apparatus 10C outputs various control signals and receives various detection signals as inputs via the I/O. For example, the control device outputs a control signal to the liquid level adjusting pump 340, the solenoid valve 370, and the like.

### <<<Overall processing of control device 66>>>

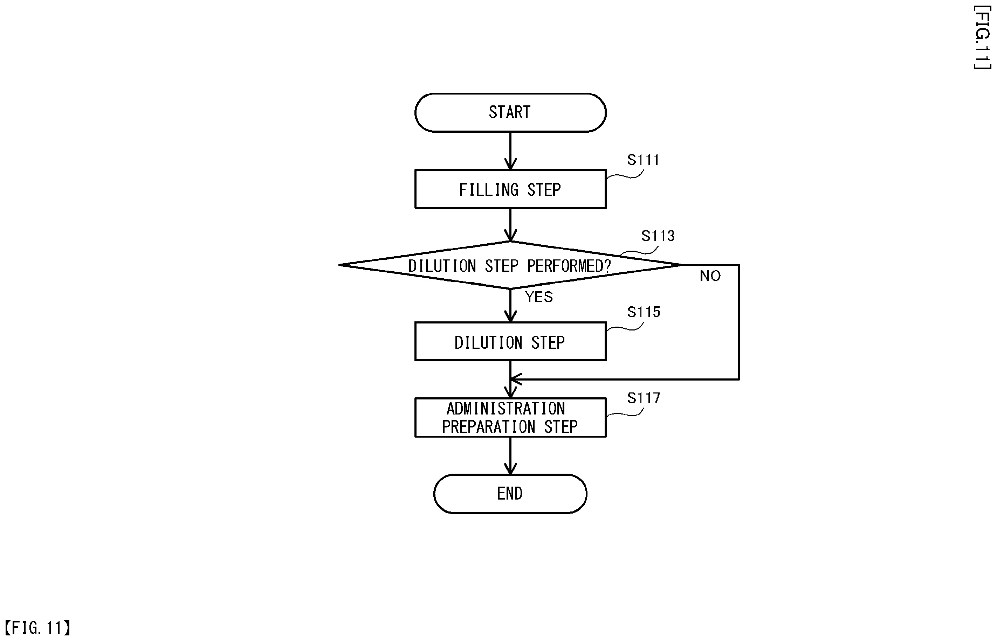

Fig. 11 is a flowchart illustrating overall processing of the control device 66. As described above, in the medicinal liquid supply apparatuses 10A to 10C, the filling step, the dilution step, and the administration preparation step are sequentially performed under control of the control device 66. In particular, when it is not necessary to dilute heparin, by operating an input portion (Fig. 10), an operator can cause the process to proceed from the filling step to the administration preparation step without performing the dilution step.

The processor of the control device 66 performs the filling step (step S111).

If heparin is diluted in step S113 (YES), the processor of the control device 66 performs the dilution step (step S115).

If heparin is not diluted in step S113 (NO) or after the dilution step is performed, the processor of the control device 66 performs the administration preparation step (step S117).

### <<<<Range of embodiments>>>>

The first to third embodiments have been described hereinabove. However, the description and drawings forming a portion of this disclosure should not be understood as being limited. Various embodiments and the like not described herein are included.

In the examples described in the first to third embodiments, heparin (unfractionated heparin) is used as an example of an anticoagulant. However, the anticoagulant is not limited to unfractionated heparin, and low molecular weight heparin, argatroban, nafamostat mesilate, and the like can be used. These can be appropriately selected and used according to characteristics such as a process of action, a half-life, and side effects.

### <<<<Aspects of invention>>>

### <<First aspect>>

A first aspect of the medicinal liquid supply apparatus provides a medicinal liquid supply apparatus that supplies a medicinal liquid to a blood circuit (for example, the arterial side blood circuit 30 or the venous side blood circuit 40) in a blood purification apparatus that purifies blood (for example, the dialysis device main body 60). The medicinal liquid supply apparatus includes
a composition-holding portion (for example, the pipette 110 or the pipette 310) into which an anticoagulant (for example, heparin) contained in a container can be introduced from the container and which holds a composition containing at least the introduced anticoagulant; and
a connecting portion (for example, the medicinal liquid line 100, 200, or 300) that connects the composition-holding portion to the blood circuit, and allows the composition to be led out from the composition-holding portion to the blood circuit.

The medicinal liquid supply apparatus is an apparatus that supplies a medicinal liquid to the blood circuit. The blood purification apparatus that purifies blood includes the blood circuit. The medicinal liquid supply apparatus includes the composition-holding portion and the connecting portion.

An anticoagulant can be introduced into the composition-holding portion. That is, an anticoagulant can be introduced into the composition-holding portion. An anticoagulant is contained in a container. The container is generally used to contain an anticoagulant. The container is generally distributed to contain an anticoagulant. The container has a shape, a size, and the like for containing an anticoagulant.

The phrase "can be introduced" means that not only a state in which an anticoagulant is introduced into the composition-holding portion but also a preliminary situation and a potential situation for introduction are included in the medicinal liquid supply apparatus according to the first aspect even in a state in which the anticoagulant is not introduced into the composition-holding portion.

For example, a situation in which the composition-holding portion can inevitably touch the anticoagulant in the container even in a state in which the anticoagulant in the container and the composition-holding portion are separated from each other corresponds to this. In addition, even when the anticoagulant in the container is in contact with the composition-holding portion, unless a drive portion such as a pump is driven, the anticoagulant is not introduced into the composition-holding portion. However, if the drive portion is driven, the anticoagulant is immediately introduced into the composition-holding portion. Such a situation or the like also corresponds to that.

As described above, the phrase "can be introduced" means also including a preliminary situation and a potential situation for introducing the anticoagulant into the composition-holding portion from the container.

The composition-holding portion holds a composition. The composition contains at least an anticoagulant introduced into the composition-holding portion. The composition may contain only the anticoagulant or may contain the anticoagulant and other components.

An anticoagulant can be introduced into the composition-holding portion, and the composition-holding portion can hold a composition containing at least the introduced anticoagulant.

The connecting portion connects the composition-holding portion to the blood circuit. The composition-holding portion is connected to the blood circuit by the connecting portion. The connecting portion can lead out the composition from the composition-holding portion to the blood circuit. That is, the composition held in the composition-holding portion can be led out from the composition-holding portion to the blood circuit via the connecting portion.

The phrase "can be led out" means that not only a state in which the composition is led out to the blood circuit but also a preliminary situation and a potential situation for lead-out are included in the medicinal liquid supply apparatus according to the first aspect even in a state in which the composition is not led out to the blood circuit.

For example, a situation in which the composition-holding portion can be inevitably connected to the blood circuit even in a state in which the composition-holding portion is disconnected from the blood circuit corresponds to this. In addition, even when the composition-holding portion is connected to the blood circuit, unless a member such as a pump or a valve is brought into a lead-out state, the composition is not led out to the blood circuit. However, if the member is brought into the lead-out state, the composition is immediately led out to the blood circuit. Such a situation or the like corresponds to that.

As described above, the phrase "can be led out" means also including a preliminary situation and a potential situation for leading out the composition to the blood circuit.

Since an anticoagulant contained in a generally used or distributed container can be introduced into the composition-holding portion, it is possible to prevent transfer of the anticoagulant into another container and disposal of the anticoagulant. It is possible to prevent operation from being complicated and to prevent the anticoagulant from being wasted.

### <<Second aspect>>

A second aspect further includes, in the first aspect, detecting a liquid level of the composition held in the composition-holding portion using a liquid level detection device (for example, the liquid level detection device 150).

Since the liquid level of the composition is detected by the liquid level detection device, the amount of the anticoagulant introduced into the composition-holding portion and the amount of the composition remaining in the composition-holding portion can be acquired, and the medicinal liquid supply apparatus can be appropriately controlled. The blood purification apparatus may include the liquid level detection device, or the medicinal liquid supply apparatus or another apparatus may include the liquid level detection device. The blood purification apparatus, the medicinal liquid supply apparatus, or the like only needs to be able to perform control based on the liquid level of the composition detected by the liquid level detection device.

### <<Third aspect>>

A third aspect further includes, in the first or second aspect, detecting an empty state of the composition in the composition-holding portion using an empty detection device (for example, the empty detection device 160).

Since it is possible to detect an empty state of the composition by the empty detection device, it is possible to appropriately determine whether or not the anticoagulant may be introduced into the composition-holding portion, whether or not lead-out of the composition should be stopped, and the like. The blood purification apparatus may include the empty detection device, or the medicinal liquid supply apparatus or another apparatus may include the empty detection device. The blood purification apparatus, the medicinal liquid supply apparatus, or the like only needs to be able to perform control based on a detection result detected by the empty detection device.

### <<Fourth aspect>>

According to a fourth aspect, in any one of the first to third aspects, a diluent (for example, a priming liquid) can be introduced into the composition-holding portion, and the composition diluted with the introduced diluent can be held in the composition-holding portion.

Not only the anticoagulant but also the diluent can be introduced into the composition-holding portion. By introducing the diluent into the anticoagulant introduced into the composition-holding portion, the anticoagulant can be diluted in the composition-holding portion, and the diluted anticoagulant can be held as a composition in the composition-holding portion.

The phrase "can be introduced" means that not only a state in which the diluent is introduced into the composition-holding portion but also a preliminary situation and a potential situation for introduction are included in the medicinal liquid supply apparatus according to the fourth aspect even in a state in which the diluent is not introduced into the composition-holding portion.

For example, a situation in which the composition-holding portion can be inevitably connected to the blood circuit even in a state in which the composition-holding portion is disconnected from the blood circuit corresponds to this. In addition, even when the composition-holding portion is connected to the blood circuit, unless a member such as a pump or a valve is brought into an introduction state, the diluent is not introduced into the composition-holding portion. However, if the member is brought into the introduction state, the diluent is immediately introduced into the composition-holding portion. Such a situation or the like corresponds to that.

As described above, the phrase "can be introduced" means also including a preliminary situation and a potential situation for introducing the diluent into the composition-holding portion from the blood circuit.

The diluent can be introduced into the composition-holding portion from the blood circuit, for example, via the connecting portion. The path of introduction of the diluent is not limited thereto, and may be any path as long as the diluent can be introduced into the composition-holding portion.

### <<Fifth aspect>>

A fifth aspect further includes: in any one of the first to fourth aspects, a first communication portion (for example, the extending portion 113 or the first lower end opening 316) that causes the container and the composition-holding portion to communicate with each other, and allows an anticoagulant contained in the container to be introduced into the composition-holding portion; and a second communication portion (for example, the lower end opening 116 or the second lower end opening 318) that causes the composition-holding portion and the connecting portion to communicate with each other, and allows the composition held in the composition-holding portion to be led out to the connecting portion.

A medicinal liquid supply apparatus according to the fifth aspect includes the first communication portion and the second communication portion.

The first communication portion causes the container and the composition-holding portion to communicate with each other. The first communication portion allows an anticoagulant contained in the container to be introduced into the composition-holding portion. The anticoagulant contained in the container can be introduced into the composition-holding portion via the first communication portion.

Note that meaning of the phrase "can be introduced" in the fifth aspect is also similar to that of the phrase "can be introduced" in the first aspect.

The second communication portion causes the composition-holding portion and the connecting portion to communicate with each other. The second communication portion allows the composition held in the composition-holding portion to be led out to the connecting portion. The composition held in the composition-holding portion can be led out to the connecting portion via the second communication portion.

Note that meaning of the phrase "can be led out" in the fifth aspect is also similar to that of the phrase "can be led out" in the first aspect.

By separately providing a path through which the anticoagulant flows and a path through which the composition or the diluent flows with the composition-holding portion interposed therebetween, a state such as a concentration or cleanliness of the anticoagulant contained in the container can be kept constant.

### <<Sixth aspect>>

According to a sixth aspect, in any one of the first to fifth aspects, the second communication portion has
a diluent introduction state in which a diluent is introduced into the composition-holding portion and
a composition lead-out state in which the composition is led out to the connecting portion.

The second communication portion can be shared by both the diluent introduction state and the composition lead-out state. The diluent introduction state is a state in which the diluent is introduced into the composition-holding portion via the connecting portion. The composition lead-out state is a state in which the composition is led out to the connecting portion via the connecting portion. The configuration can be simplified by sharing the second communication portion.

### <<Seventh aspect>>

According to a seventh aspect, in any one of the first to sixth aspects,
the blood circuit further includes a diaphragm pump (for example, the diaphragm pump 130) that controls a flow of liquid in the blood circuit by deformation of a diaphragm.

Since a flow of liquid in the blood circuit is controlled using the diaphragm pump included in the blood circuit, the diluent can be introduced into the composition-holding portion or the composition can be led out to the connecting portion without separately disposing a drive device for controlling the flow of the liquid. The configuration of the medicinal liquid supply apparatus can be simplified by sharing the diaphragm pump.

### <<Eighth aspect>>

An eighth aspect further includes, in any one of the first to seventh aspects,
a dropping chamber (for example, the dropping chamber 210) that separates liquid continuously flowing down into independent droplets and drops the separated droplets by action of gravity,
in which the composition dropped from the dropping chamber is detected using a dropping sensor (for example, the intravenous drip sensor 220).

Since the composition can be led out from the composition-holding portion to the blood circuit using a member that is normally included or commonly used in a dialysis facility or the like, the medicinal liquid supply apparatus can be easily and simply configured. The blood purification apparatus may include the dropping sensor, or the medicinal liquid supply apparatus or another apparatus may include the dropping sensor. The blood purification apparatus, the medicinal liquid supply apparatus, or the like only needs to be able to perform control based on a detection result detected by the dropping sensor.

### <<Ninth aspect>>

According to a ninth aspect, in any one of the first to eighth aspects,
an extending portion (for example, the extending portion 313) away from the composition-holding portion has a diaphragm pump (for example, diaphragm pump 330) that controls a flow of air in the extending portion by deformation of a diaphragm.

The term "flowable" means that not only a state in which air is flowing but also a preliminary situation and a potential situation for flowing are included in the medicinal liquid supply apparatus according to the ninth aspect even in a state in which air is not flowing.

For example, unless a member such as a pump or a valve becomes flowable, air does not flow in the extending portion. However, if the member becomes flowable, air immediately flows in the extending portion. Such a situation or the like corresponds to that.

As described above, the term "flowable" means also including a preliminary situation and a potential situation for air to flow in the extending portion.

Even when the blood circuit does not include a drive device such as a diaphragm pump, introduction of an anticoagulant, introduction of a diluent, and lead-out of a composition can be performed by separately adding the diaphragm pump to the blood circuit.

The extending portion may be included in the medicinal liquid supply apparatus, or does not have to be included therein.

### <<Tenth aspect>>

According to a tenth aspect, in any one of the first to ninth aspects,
the first communication portion has a protruding portion (for example, the extending portion 113) having a long and cylindrical shape, and
the protruding portion has an opening (for example, the upper end opening 114) that can be inserted into a through hole of the container and is formed at a distal end farthest from the composition-holding portion.

The first communication portion has a protruding portion protruding from the composition-holding portion. The protruding portion has an opening. The opening can be inserted into a through hole of the container. The opening is formed at a distal end farthest from the composition-holding portion.

The phrase "can be inserted" means that not only a state in which the opening is inserted into the through hole but also a preliminary situation and a potential situation for insertion are included in the medicinal liquid supply apparatus according to the tenth aspect even in a state in which the opening is not inserted into the through hole.

For example, a situation in which the opening can be inevitably inserted into the through hole even in a state in which the opening is away from the through hole corresponds to this.

As described above, the phrase "can be inserted" means also including a preliminary situation and a potential situation for inserting the opening into the through hole.

By locating the protruding portion inside the container through the through-hole, the anticoagulant contained in the container can be immediately introduced into the composition-holding portion via the opening, the anticoagulant can be used without processing the container or transferring the anticoagulant into another container, and operation can be simplified.

Note that the outer diameter of the protruding portion preferably decreases as it goes away from the composition-holding portion. It is possible to correspond to the sizes of various through holes of the container, and it is possible to increase the number of types of containers in which the first communication portion can be used.

### <<Eleventh aspect>>

An eleventh aspect provides, in any one of the first to tenth aspects,
a medicinal liquid supply method for supplying a medicinal liquid to a blood circuit (for example, the arterial side blood circuit 30 or the venous side blood circuit 40) in a blood purification apparatus (for example, the dialysis device main body 60) that purifies blood. The medicinal liquid supply method includes
an anticoagulant introduction step of introducing an anticoagulant (for example, heparin) contained in a container from the container into a composition-holding portion (for example, the pipette 110 or the pipette 310);
a composition holding step of holding a composition containing at least the anticoagulant introduced into the composition-holding portion in the composition-holding portion; and
a composition lead-out step of leading out the composition from the composition-holding portion to the blood circuit via a connecting portion (for example, the medicinal liquid line 100, 200, or 300) connecting the composition-holding portion to the blood circuit.

Since an anticoagulant contained in a generally used or distributed container can be introduced into the composition-holding portion, it is possible to prevent transfer of the anticoagulant into another container and disposal of the anticoagulant. It is possible to prevent operation from being complicated and to prevent the anticoagulant from being wasted.

### <<Twelfth aspect>>

A twelfth aspect further includes, in any one of the first to eleventh aspects, a composition liquid level detection step of detecting a liquid level of the composition held in the composition-holding portion.

Since the liquid level of the composition is detected by the liquid level detection device, the amount of the anticoagulant introduced into the composition-holding portion and the amount of the composition remaining in the composition-holding portion can be acquired, and the medicinal liquid supply apparatus can be appropriately controlled.

### <<Thirteenth aspect>>

A thirteenth aspect further includes, in any one of the first to twelfth aspects, an empty detection step of detecting an empty state of the composition in the composition-holding portion.

Since it is possible to detect an empty state of the composition by the empty detection device, it is possible to appropriately determine whether or not the anticoagulant may be introduced into the composition-holding portion, whether or not lead-out of the composition should be stopped, and the like.

### <<Fourteenth aspect>>

A fourteenth aspect further includes: in any one of the first to thirteenth aspects, a diluent introduction step of introducing a diluent (for example, a priming liquid) into the composition-holding portion; and
a dilution holding step of holding the composition diluted with the introduced diluent in the composition-holding portion.

Not only the anticoagulant but also the diluent can be introduced into the composition-holding portion. By introducing the diluent into the anticoagulant introduced into the composition-holding portion, the anticoagulant can be diluted in the composition-holding portion, and the diluted anticoagulant can be held as a composition in the composition-holding portion.

### <<Fifteenth aspect>>

A fifteenth aspect further includes: in any one of the first to fourteenth aspects,
an introduction step of introducing an anticoagulant contained in the container into the composition-holding portion via a first communication portion (for example, the extending portion 113 or the first lower end opening 316) that causes the container and the composition-holding portion to communicate with each other; and
a lead-out step of leading out the composition held in the composition-holding portion to the connecting portion via a second communication portion (for example, the lower end opening 116 or the second lower end opening 318) that causes the composition-holding portion and the connecting portion to communicate with each other.

By separately providing a path through which the anticoagulant flows and a path through which the composition or the diluent flows with the composition-holding portion interposed therebetween, a state such as a concentration or cleanliness of the anticoagulant contained in the container can be kept constant.

### <<Sixteenth aspect>>

A sixteenth aspect further includes: in any one of the first to fifteenth aspects,
a diluent introduction step of introducing a diluent into the composition-holding portion via the second communication portion; and
a composition lead-out step of leading out the composition to the connecting portion via the second communication portion.

The second communication portion can be shared by both the diluent introduction step and the composition lead-out step. The diluent introduction step is a step of introducing a diluent into the composition-holding portion via the connecting portion. The composition lead-out step is a step of leading out the composition to the connecting portion via the connecting portion. The configuration can be simplified by sharing the second communication portion.

### <<Seventeenth aspect>>

A seventeenth aspect further includes, in any one of the first to sixteenth aspects, a liquid flow control step of controlling a flow of liquid in the blood circuit by deformation of a diaphragm in a diaphragm pump (for example, the diaphragm pump 130).

Since a flow of liquid in the blood circuit is controlled using the diaphragm pump included in the blood circuit, the diluent can be introduced into the composition-holding portion or the composition can be led out to the connecting portion without separately disposing a drive device for controlling the flow of the liquid. The configuration of the medicinal liquid supply apparatus can be simplified by sharing the diaphragm pump.

### <<Eighteenth aspect>>

An eighteenth aspect further includes: in any one of the first to seventeenth aspects,
a dropping step of separating the composition continuously flowing down into independent droplets and dropping the separated drops by action of gravity; and
a dropping composition detection step of detecting the dropped composition.

Since the composition can be led out from the composition-holding portion to the blood circuit using a member that is normally included or commonly used in a dialysis facility or the like, the medicinal liquid supply apparatus can be easily and simply configured.

### <<Nineteenth aspect>>

A nineteenth aspect further includes, in any one of the first to eighteenth aspects, a liquid flow control step of controlling, by deformation of a diaphragm in a diaphragm pump (for example, the diaphragm pump 330) disposed in an extending portion which is away from the composition-holding portion and through which air can flow, a flow of liquid in the extending portion.

Even when the blood circuit does not include a drive device such as a diaphragm pump, introduction of an anticoagulant, introduction of a diluent, and lead-out of a composition can be performed by separately adding the diaphragm pump to the blood circuit.

### INDUSTRIAL APPLICABILITY

Provided is a medicinal liquid supply apparatus which is not limited by a packaging material of a medicinal liquid such as heparin, and can be used for treatment to be performed a plurality of times with the medicinal liquid in a unit of packaging.

### CROSS-REFERENCE TO RELATED APPLICATIONS

The present application claims priority based on Japanese Patent Application No. 2023-209695 filed with the Japan Patent Office on December 12, 2023, the entire disclosure of which is entirely incorporated herein by reference.

### REFERENCE SIGNS LIST

- 10A, 10B, 10C: Medicinal liquid supply apparatus
- 20: Dialyzer
- 60: Dialysis device main body
- 70: Blood purification apparatus
- 110: Pipette
- 130: Diaphragm pump
- 140: Liquid level adjusting pump

## Claims

1. A medicinal liquid supply apparatus that supplies a medicinal liquid to a blood circuit in a blood purification apparatus that purifies blood, the medicinal liquid supply apparatus comprising:
a composition-holding portion into which an anticoagulant contained in a container can be introduced from the container and which holds a composition containing at least the introduced anticoagulant; and
a connecting portion that connects the composition-holding portion to the blood circuit, and allows the composition to be led out from the composition-holding portion to the blood circuit.

2. The medicinal liquid supply apparatus according to claim 1, wherein a liquid level of the composition held in the composition-holding portion is detected using a liquid level detection device.

3. The medicinal liquid supply apparatus according to claim 1, wherein an empty state of the composition in the composition-holding portion is detected using an empty detection device.

4. The medicinal liquid supply apparatus according to claim 1, wherein
a diluent can be introduced into the composition-holding portion, and
the composition diluted with the introduced diluent can be held in the composition-holding portion.

5. The medicinal liquid supply apparatus according to claim 1, further comprising:
a first communication portion that causes the container and the composition-holding portion to communicate with each other, and allows an anticoagulant contained in the container to be introduced into the composition-holding portion; and
a second communication portion that causes the composition-holding portion and the connecting portion to communicate with each other, and allows the composition held in the composition-holding portion to be led out to the connecting portion.

6. The medicinal liquid supply apparatus according to claim 5, wherein
the second communication portion has:
a diluent introduction state in which a diluent is introduced into the composition-holding portion; and
a composition lead-out state in which the composition is led out to the connecting portion.

7. The medicinal liquid supply apparatus according to claim 1, wherein the blood circuit further comprises a diaphragm pump that controls a flow of liquid in the blood circuit by deformation of a diaphragm.

8. The medicinal liquid supply apparatus according to claim 1, further comprising
a dropping chamber that separates the composition continuously flowing down into independent droplets and drops the separated droplets by action of gravity, wherein
the composition dropped from the dropping chamber is detected using a dropping sensor.

9. The medicinal liquid supply apparatus according to claim 1, wherein an extending portion away from the composition-holding portion has a diaphragm pump that controls a flow of air in the extending portion by deformation of a diaphragm.

10. The medicinal liquid supply apparatus according to claim 5, wherein
the first communication portion has a protruding portion having a long and cylindrical shape, and
the protruding portion has an opening that can be inserted into a through hole of the container and is formed at a distal end farthest from the composition-holding portion.

11. A medicinal liquid supply method for supplying a medicinal liquid to a blood circuit in a blood purification apparatus that purifies blood, the method comprising:
an anticoagulant introduction step of introducing an anticoagulant contained in a container from the container into a composition-holding portion;
a composition holding step of holding a composition containing at least the anticoagulant introduced into the composition-holding portion in the composition-holding portion; and
a composition lead-out step of leading out the composition from the composition-holding portion to the blood circuit via a connecting portion connecting the composition-holding portion to the blood circuit.

12. The medicinal liquid supply method according to claim 11, further comprising a composition liquid level detection step of detecting a liquid level of the composition held in the composition-holding portion.

13. The medicinal liquid supply method according to claim 11, further comprising an empty detection step of detecting an empty state of the composition in the composition-holding portion.

14. The medicinal liquid supply method according to claim 11, further comprising: a diluent introduction step of introducing a diluent into the composition-holding portion; and
a dilution holding step of holding the composition diluted with the introduced diluent in the composition-holding portion.

15. The medicinal liquid supply method according to claim 11, further comprising:
an introduction step of introducing an anticoagulant contained in the container into the composition-holding portion via a first communication portion that causes the container and the composition-holding portion to communicate with each other; and
a lead-out step of leading out the composition held in the composition-holding portion to the connecting portion via a second communication portion that causes the composition-holding portion and the connecting portion to communicate with each other.

16. The medicinal liquid supply method according to claim 15, further comprising:
a diluent introduction step of introducing a diluent into the composition-holding portion via the second communication portion; and
a composition lead-out step of leading out the composition to the connecting portion via the second communication portion.

17. The medicinal liquid supply method according to claim 11, further comprising a liquid flow control step of controlling a flow of liquid in the blood circuit by deformation of a diaphragm in a diaphragm pump.

18. The medicinal liquid supply apparatus according to claim 11, further comprising:
a dropping step of separating the composition continuously flowing down into independent droplets and dropping the separated drops by action of gravity; and
a dropping composition detection step of detecting the dropped composition.

19. The medicinal liquid supply method according to claim 11, further comprising a liquid flow control step of controlling a flow of air in an extending portion which is away from the composition-holding portion and through which air can flow by deformation of a diaphragm in a diaphragm pump disposed in the extending portion.
